# DEMANDE DE BREVET EUROPEEN

(11) **EP 0 731 168 A1**
(43) Date de publication de la demande: **11.09.1996**
(21) Numéro de dépôt: 96420070.3
(22) Date de dépôt: 05.03.1996
(51) Int. Cl.: C12N 15/48, C07K 14/15, C12Q 1/68, C12N 15/11, A61K 35/76, C12N 7/02, A61K 39/21, G01N 33/569

(54) **Virus MSRV-1 et agent pathogène et/ou infectant MSRV-2 associés à la polyarthrite rhumatoide**

(30) Priorité: 09.03.1995 FR 9502960
(71) Demandeur: BIO MERIEUX, F-69280 Marcy l'Etoile (FR)
(72) Inventeur: Perron, Hervé, 69005 Lyon (FR); Bedin, Frédéric, 69002 Lyon (FR); Mandrand, Bernard, 69100 Villeurbanne (FR); Beseme, Frédéric, 38090 Villefontaine (FR); Mallet, François, 69100 Villeurbanne (FR)
(74) Mandataire: Guerre, Dominique

(57) **Abrégé**

L'invention concerne l'utilisation d'un matériel viral, à l'état purifié ou isolé, possédant une activité transcriptase inverse, apparenté à une famille d'éléments rétroviraux endogènes, et/ou d'un agent pathogène et/ou infectant, à l'état purifié ou isolé, différent du matériel viral précité, chacun étant issu d'une souche virale possédant une activité transcriptase inverse, choisie parmi les souches dénommées respectivement POL-2, déposée le 22.07.1992 auprès de l'ECACC sous le numéro d'accès V92072202, et MS7PG déposée le 08.01.93 auprès de l'ECACC sous le numéro d'accès V93010816, et parmi leurs souches variantes consistant en des virus comprenant au moins un antigène qui est reconnu par au moins un anticorps dirigé contre au moins un antigène correspondant de l'un ou l'autre des virus des souches virales POL-2 et MS7PG précitées, pour obtenir une composition diagnostique, prophylactique ou thérapeutique pour détecter, prévenir ou traiter une infection par ledit matériel viral et/ou ledit agent pathogène et/ou infectieux, associés à la polyarthrite rhumatoïde.

## Description

La polyarthrite rhumatoïde (PR) est le plus fréquent des rhumatismes inflammatoires humains et sa prévalence est particulièrement élevée : 3% **(1)**. Le diagnostic en est malaisé à son début : son hétérogénéité clinique ainsi que les symptômes extra-articulaires associés à des anomalies immunologiques parfois importantes, viennent compliquer le tableau. Il s'agit d'une maladie d'étiologie encore inconnue qui est classée dans la catégorie des maladies "autoimmunes" du fait de l'existence de réactions immunologiques exacerbées contre des auto-antigènes.

Une étiologie virale et/ou bactérienne de la PR a été régulièrement invoquée mais la recherche d'un agent causal de la PR n'a pas abouti à ce jour **(2)**.

Les concepts définissant la PR comme une maladie auto-immune et ceux qui étayent une hypothèse étiologique virale, voire bactérienne, peuvent maintenant se rejoindre dans la compréhension des différents mécanismes par lesquels un micro-organisme peut induire une réaction auto-immune chez l'hôte infecté, comme cela a été décrit par Fujinami R.S. et Oldstone M.B.A. **(3)**. Des molécules d'origine bactérienne ou virale, voire rétrovirale endogène, sont connues pour posséder des propriétés dites superantigéniques **(4,5)**. Leurs propriétés particulières de stimulation directe des lymphocytes T, spécifiques d'antigènes différents, par liaison avec la région Vβ de certains récepteurs "T", ont fait évoquer l'hypothèse que de telles molécules soient intimement associées au processus étiopathogénique des pathologies autoimmunes **(6)**. Pourtant, aucun agent pathogène spécifique, bactérien ou viral, et éventuellement producteur de superantigène, n'a encore été clairement associé à la polyarthrite rhumatoïde.

Les travaux de la Demanderesse, dans la recherche d'une étiologie de la PR l'ont conduite aujourd'hui à la découverte de l'existence de deux agents pathologiques et/ou infectants, associés, indépendamment ou ensemble, aux états pathologiques de la PR.

Les techniques de culture et de détection de matériel rétroviral mises en oeuvre dans les travaux réalisés par la Demanderesse sur un agent associé à la sclérose en plaques (SEP) et décrits dans les demandes de brevet français 92 04322, 92 13447, 92 13443, 92 01529, 94 01530, 94 01531, 94 01532 et dans la publication de H. PERRON et coll. **(7)** (dont le contenu est incorporé par référence à la présente description), ont permis de mettre en évidence, de manière totalement inattendue, l'identité entre des agents associés à la SEP et ceux qui font l'objet de la présente invention, associés à la PR.

Etant donné que, dans la PR, le processus a lieu au niveau de l'articulation et implique plus particulièrement la synoviale, on a réalisé des cultures de cellules dérivées de liquide synovial ponctionné dans des articulations affectées de patients atteints de PR. A partir d'une telle culture, on a pu obtenir des cellules de type fibroblastique qui ont proliféré in vitro, permettant ainsi de réaliser quelques passages. Les milieux de culture correspondants ont été utilisés pour une recherche d'activité transcriptase inverse associée à des particules infectantes concentrées par ultracentrifugation puis purifiées sur gradient isopycnique. L'utilisation des conditions de détection mises au point pour la souche LM7 issue de SEP **(7)**, a permis de détecter une activité transcriptase inverse significative dans quelques fractions individualisées d'un tel gradient. Par la suite, l'analyse des séquences nucléiques amplifiées dans ces fractions par la technique PCR (Polymerase Chain Reaction), avec des amorces dégénérées analogues aux séquences consensus des enzymes à activité transcriptase inverse **(8)** a révélé la présence de séquences significatives identiques aux séquences des agents pathogènes et/ou infectieux MSRV1 et MSRV2, précédemment identifiées dans les cultures issues de cas de sclérose en plaques.

Ainsi, les différents objets de la présente invention sont les suivants:
i) l'utilisation d'un matériel viral, à l'état purifié ou isolé, possédant une activité transcriptase inverse, apparenté à une famille d'éléments rétroviraux endogènes, tel qu'issu d'une souche virale possédant une activité transcriptase inverse, choisie parmi les souches dénommées respectivement POL-2, déposée le 22.07.1992 auprès de l'ECACC sous le numéro d'accès V92072202, et MS7PG déposée le 08.01.93 auprès de l'ECACC sous le numéro d'accès V93010816, et parmi leurs souches variantes, les souches variantes consistant en des virus comprenant au moins un antigène qui est reconnu par au moins un anticorps dirigé contre au moins un antigène correspondant de l'un ou l'autre des virus des souches virales POL-2 et MS7PG précitées, pour obtenir une composition diagnostique, prophylactique ou thérapeutique pour détecter, prévenir ou traiter une infection par ledit matériel viral ou une réactivation dudit matériel viral, associé à la polyarthrite rhumatoïde,
ii) l'utilisation d'un matériel viral, à l'état purifié ou isolé, possédant une activité transcriptase inverse, apparenté à une famille d'éléments rétroviraux endogènes, tel que produit par une lignée cellulaire choisie parmi les lignées dénommées respectivement PLI-2 déposée le 22.07.1992 auprès de l'ECACC sous le numéro d'accès 92072201, et LM7PC déposée le 08.01.93 auprès de l'ECACC sous le numéro d'accès 93010817, ou par toute culture cellulaire infectée susceptible de produire un virus comprenant au moins un antigène qui est reconnu par au moins un anticorps dirigé contre au moins un antigène correspondant de l'un ou l'autre des virus produits par les lignées PLI-2 et LM7PC précitées, pour obtenir une composition diagnostique, prophylactique ou thérapeutique pour détecter, prévenir ou traiter une infection par ledit matériel viral ou une réactivation dudit matériel viral, associé à la polyarthrite rhumatoïde,
iii) l'utilisation d'un matériel viral dont le génome comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 leurs séquences complémentaires, et leurs séquences équivalentes, notamment les séquences nucléotidiques présentant, pour toute suite de 100 monomères contigus, au moins 50 % et préférentiellement au moins 70 % d'homologie avec une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 et leurs séquences complémentaires, pour obtenir une composition diagnostique, prophylactique ou thérapeutique pour détecter, prévenir ou traiter une infection par ledit matériel viral ou une réactivation dudit matériel viral, associé à la polyarthrite rhumatoïde,
iv) l'utilisation d'un matériel rétroviral, dont le gène *pol* de son génome comprend une séquence nucléotidique équivalente, et notamment présentant au moins 50% d'homologie, de préférence au moins 65% d'homologie, avec une séquence nucléotidique appartenant au gène *pol* du génome du rétrovirus ERV-9 ou HSERV-9, pour obtenir une composition diagnostique, prophylactique ou thérapeutique, pour détecter, prévenir ou traiter une infection par ledit matériel viral ou une réactivation dudit matériel viral, associé à la polyarthrite rhumatoïde,
v) l'utilisation d'un matériel rétroviral dont le gène *pol* de son génome code pour une séquence peptidique 5 présentant au moins 50%, et de préférence au moins 70% d'homologie avec une séquence peptidique codée par le gène *pol* du génome du rétrovirus ERV-9 ou HSERV-9, pour obtenir une composition diagnostique, prophylactique ou thérapeutique, pour détecter, prévenir ou traiter une infection par ledit matériel viral ou une réactivation dudit matériel viral, associé à la polyarthrite rhumatoïde,
vi) l'utilisation d'un matériel rétroviral dont le gène *pol* de son génome code pour une séquence peptidique présentant, pour toute suite contigue d'au moins 30 acides aminés, au moins 50% et de préférence au moins 70% d'homologie avec une séquence peptidique codée par une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, et leurs séquences complémentaires, pour obtenir une composition diagnostique, prophylactique ou thérapeutique, pour détecter, prévenir ou traiter une infection par ledit matériel viral ou une réactivation dudit matériel viral, associé à la polyarthrite rhumatoïde,
vii) l'utilisation d'un fragment nucléotidique, dont la séquence nucléotidique comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, leurs séquences complémentaires, et leurs séquences équivalentes, notamment les séquences nucléotidiques présentant, pour toute suite de 100 monomères contigus, au moins 50%, et de préférence au moins 70% d'homologie, avec une séquence choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 et leurs séquences complémentaires, pour obtenir une composition diagnostique, prophylactique ou thérapeutique, pour détecter, prévenir ou traiter une infection par ledit matériel viral ou une réactivation dudit matériel viral, associé à la polyarthrite rhumatoïde,
viii) l'utilisation d'une amorce spécifique comprenant une séquence nucléotidique identique ou équivalente à au moins une partie de la séquence nucléotidique d'un fragment défini en vii), notamment une séquence nucléotidique présentant, pour toute suite de 10 monomères contigus, au moins 70% d'homologie avec au moins une partie dudit fragment, pour l'amplification par polymérisation d'un ARN ou d'un ADN d'un matériel viral associé à la polyarthrite rhumatoïde ; selon une utilisation préférentielle, l'amorce comprend une séquence nucléotidique choisie parmi SEQ ID N016, SEQ ID N017, SEQ ID N018, SEQ ID N019, SEQ ID N020, SEQ ID N021, SEQ ID N022, SEQ ID N023, SEQ ID N024, SEQ ID N025, SEQ ID N026, SEQ ID N031, SEQ ID N032, SEQ ID N033, SEQ ID N047, SEQ ID N048, SEQ ID N049 et leurs séquences complémentaires,
ix) l'utilisation d'une sonde comprenant une séquence nucléotidique identique ou équivalente à au moins une partie de la séquence nucléotidique d'un fragment défini en vii), notamment une séquence nucléotidique présentant, pour toute suite de 10 monomères contigus, au moins 70% d'homologie avec au moins une partie dudit fragment pour obtenir une composition pour détecter, séparer, ou identifier, dans un échantillon biologique, un matériel viral ou une réactivation dudit matériel viral, associé à la polyarthrite rhumatoïde ; selon une utilisation préférentielle, la sonde comprend une séquence nucléotidique choisie parmi SEQ ID N03, SEQ ID N04, SEQ ID N05, SEQ ID N06, SEQ ID N07, SEQ ID N016, SEQ ID N017, SEQ ID N018, SEQ ID N019, SEQ ID N020, SEQ ID N021, SEQ ID N022, SEQ ID N023, SEQ ID N024, SEQ ID N025, SEQ ID N026, SEQ ID N031, SEQ ID N032, SEQ ID N033, SEQ ID N047, SEQ ID N048, SEQ ID N049 et leurs séquences complémentaires,
x) l'utilisation d'un agent pathogène et/ou infectant, à l'état purifié ou isolé, différent du matériel viral défini à l'un quelconque des points i) à vi), tel qu'issu d'une souche virale choisie parmi les souches dénommées respectivement POL-2, déposée le 22.07.1992 auprès de l'ECACC sous le numéro d'accès V92072202 et MS7PG déposée le 08.01.93 auprès de l'ECACC sous le numéro d'accès V93010816, et parmi leurs souches variantes, consistant en des agents pathogènes et/ou infectants comprenant au moins un antigène qui est reconnu par au moins un anticorps dirigé contre au moins un antigène correspondant de l'un ou l'autre des agents pathogènes et/ou infectants des souches virales POL-2 et MS7PG précitées, respectivement différents de l'un ou l'autre matériel rétroviral desdites souches, pour obtenir une composition diagnostique, prophylactique ou thérapeutique, pour détecter, prévenir ou traiter une infection par ledit agent pathogène et/ou infectant ou une réactivation dudit agent pathogène et/ou infectant, associé à la polyarthrite rhumatoïde,
xi) l'utilisation d'un agent pathogène et/ou infectant, à l'état purifié ou isolé, différent du matériel viral défini à l'un quelconque des points i) à vi), tel que produit par une lignée cellulaire choisie parmi les lignées dénommées respectivement PLI-2 déposée le 22.07.1992 auprès de l'ECACC sous le numéro d'accès 92072201 et LM7PC déposée le 08.01.93 auprès de l'ECACC sous le numéro d'accès 93010817, et par toutes cultures cellulaires infectées susceptibles de produire au moins l'un ou l'autre des agents pathogènes et/ou infectants, et/ou leurs variants, ou par toute culture cellulaire infectée susceptible de produire un agent pathogène et/ou infectant comprenant au moins un antigène qui est reconnu par au moins un anticorps dirigé contre au moins un antigène correspondant de l'un ou l'autre des agents pathogènes et/ou infectants produits par les lignées PLI-2 et LM7PC précitées, pour obtenir une composition diagnostique, prophylactique ou thérapeutique, pour détecter, prévenir ou traiter une infection par ledit agent pathogène et/ou infectant ou une réactivation dudit agent pathogène et/ou infectant, associé à la polyarthrite rhumatoïde,
xii) l'utilisation d'un agent pathogène et/ou infectant comprenant un acide nucléique comprenant une séquence nucléotidique choisie parmi SEQ ID N010, SEQ ID N011, SEQ ID N012, SEQ ID N040, SEQ ID N041, leurs séquences complémentaires, et leurs séquences équivalentes, notamment les séquences nucléotidiques, présentant au moins 70% et préférentiellement au moins 90% d'homologie avec une séquence nucléotidique comprenant une séquence choisie parmi SEQ ID N010, SEQ ID N011, SEQ ID N012, SEQ ID N040, SEQ ID N041, et leurs séquences complémentaires, pour obtenir une composition diagnostique, prophylactique ou thérapeutique, pour détecter, prévenir ou traiter une infection par ledit agent pathogène et/ou infectant ou une réactivation dudit agent pathogène et/ou infectant, associé à la polyarthrite rhumatoïde,
xiii) l'utilisation d'un fragment nucléotidique, comprenant une séquence nucléotidique choisie parmi SEQ ID N010, SEQ ID N011, SEQ ID N012, SEQ ID N040, SEQ ID N041, leurs séquences complémentaires, et leurs séquences équivalentes, notamment les séquences nucléotidiques présentant, pour toute suite de 100 monomères contigus, au moins 70%, et de préférence au moins 90% d'homologie, avec une séquence choisie parmi SEQ ID N010, SEQ ID N011, SEQ ID N012, SEQ ID N040, SEQ ID N041, et leurs séquences complémentaires, pour obtenir une composition diagnostique, prophylactique ou thérapeutique pour détecter, prévenir ou traiter une infection par ledit agent pathogène et/ou infectant ou une réactivation dudit agent pathogène et/ou infectant, associé à la polyarthrite rhumatoïde,
xiv) l'utilisation d'une amorce comprenant une séquence nucléotidique identique ou équivalente à au moins une partie de la séquence nucléotidique d'un fragment défini en xiii), notamment une séquence nucléotidique présentant, pour toute suite de 10 monomères contigus, au moins 90% d'homologie avec au moins une partie dudit fragment, pour l'amplification par polymérisation d'un ARN ou d'un ADN d'un agent pathogène et/ou infectant associé à la polyarthrite rhumatoïde ; selon une utilisation avantageuse, l'amorce comprend une séquence nucléotidique choisie parmi SEQ ID N013, SEQ ID N014, SEQ ID N015, SEQ ID N027, SEQ ID N028, SEQ ID N029, SEQ ID N030, SEQ ID N034, SEQ ID N035, SEQ ID N036, SEQ ID N037, SEQ ID N044, SEQ ID N045, SEQ ID N046, et leurs séquences complémentaires,
xv) l'utilisation d'une sonde comprenant une séquence nucléotidique identique ou équivalente à au moins une partie de la séquence nucléotidique d'un fragment défini en xiii), notamment une séquence nucléotidique présentant, pour toute suite de 10 monomères contigus, au moins 90% d'homologie avec au moins une partie dudit fragment, pour obtenir une composition diagnostique, prophylactique ou thérapeutique, pour détecter, prévenir ou traiter une infection par ledit agent pathogène et/ou infectant ou une réactivation dudit agent pathogène et/ou infectant, associé à la polyarthrite rhumatoïde ; selon une utilisation avantageuse, la sonde comprend une séquence nucléotidique choisie parmi SEQ ID N010, SEQ ID N011, SEQ ID N013, SEQ ID N014, SEQ ID N015, SEQ ID N027, SEQ ID N028, SEQ ID N029, SEQ ID N030, SEQ ID N034, SEQ ID N035, SEQ ID N036, SEQ ID N037, SEQ ID N044, SEQ ID N045, SEQ ID N046 et leurs séquences complémentaires,
xvi) l'utilisation d'une association comprenant deux agents pathogènes et/ou infectants, à l'état isolé ou purifié, à savoir, un premier agent qui consiste en un virus humain, possédant une activité transcriptase inverse, et apparenté à une famille d'éléments rétroviraux endogènes, ou un variant dudit virus, et un second agent, ou un variant dudit second agent, ces deux agents pathogènes et/ou infectants étant tels que ceux issus d'une même souche virale choisie parmi les souches dénommées respectivement POL-2, déposée le 22.07.1992 auprès de l'ECACC sous le numéro d'accès V92072202 et MS7PG déposée le 08.01.93 auprès de l'ECACC sous le numéro d'accès V93010816, et parmi leurs souches variantes, pour obtenir une composition diagnostique, prophylactique ou thérapeutique pour détecter, prévenir ou traiter une infection par un premier agent pathogène et/ou infectant, et un second agent pathogène et/ou infectant, associés à la polyarthrite rhumatoïde, ou une réactivation de l'un ou l'autre dudit premier agent et dudit second agent,
xvii) l'utilisation d'une association comprenant deux agents pathogènes et/ou infectants, à l'état isolé ou purifié, à savoir, un premier agent consistant en un virus humain possédant une activité transcriptase inverse, et apparenté à une famille d'éléments rétroviraux endogènes, ou un variant dudit virus, et un second agent, ou un variant dudit second agent, ces deux agents pathogènes et/ou infectants étant tels que ceux produits par une même lignée cellulaire choisie parmi les lignées dénommées respectivement PLI-2 déposée le 22.07.1992 auprès de l'ECACC sous le numéro d'accès 92072201 et LM7PC déposée le 08.01.93 auprès de l'ECACC sous le numéro d'accès 93010817, et par toutes cultures cellulaires infectées susceptibles de produire au moins l'un ou l'autre des agents pathogènes et/ou infectants, et/ou leurs variants, pour obtenir une composition diagnostique, prophylactique ou thérapeutique pour détecter, prévenir ou traiter une infection par un premier agent pathogène et/ou infectant, et un second agent pathogène et/ou infectant, associés à la polyarthrite rhumatoïde, ou une réactivation de l'un ou l'autre dudit premier agent et dudit second agent,
xviii) l'utilisation d'une association comprenant deux agents pathogènes et/ou infectants, à l'état isolé ou purifié, à savoir un premier agent consistant en un virus, ou un variant dudit virus, dont le génome comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, leurs séquences complémentaires, et leurs séquences équivalentes, notamment les séquences nucléotidiques présentant, pour toute suite de 100 monomères contigus, au moins 50% et préférentiellement au moins 70% d'homologie avec une séquence nucléotidique choisie parmi SEQ ID N01, SEQ ID N02, SEQ ID N03, SEQ ID N04, SEQ ID N05, SEQ IDN06, SEQ ID N07, SEQ ID N08, SEQ ID N09, SEQ ID N039, SEQ ID N042, SEQ ID N043, et leurs séquences complémentaires, et un second agent pathogène et/ou infectant, dont le génome comprend une séquence nucléotidique choisie parmi SEQ ID N010, SEQ ID N011, et SEQ ID N012, SEQ ID N040, SEQ ID N041, leurs séquences complémentaires, et leurs séquences équivalentes, notamment les séquences nucléotidiques présentant, pour toute suite de 100 monomères contigus, au moins 70% et préférentiellement au moins 90% d'homologie avec une séquence nucléotidique choisie parmi SEQ ID N010, SEQ ID N011, SEQ ID N012, SEQ ID N040, SEQ ID N041, et leurs séquences complémentaires, pour obtenir une composition diagnostique, prophylactique ou thérapeutique, pour détecter, prévenir ou traiter une infection par un premier agent pathogène et/ou infectant, et un second agent pathogène et/ou infectant, associés à la polyarthrite rhumatoïde, ou une réactivation de l'un ou l'autre dudit premier agent et dudit second agent,
xix) l'utilisation d'une association de fragments nucléotidiques comprenant un premier fragment dont la séquence nucléotidique comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, leurs séquences complémentaires, et leurs séquences équivalentes, notammment les séquences nucléotidiques présentant, pour toute suite de 100 monomères contigus, au moins 50% et de préférence au moins 70% d'homologie avec une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, et leurs séquences complémentaires, et un second fragment dont la séquence nucléotidique comprend une séquence nucléotidique choisie parmi SEQ ID N010, SEQ ID N011, SEQ ID N012, SEQ ID N040, SEQ ID N041, leurs séquences complémentaires, et leurs séquences équivalentes, notammment les séquences nucléotidiques présentant, pour toute suite de 100 monomères contigus, au moins 70% et de préférence au moins 90% d'homologie avec une séquence nucléotidique choisie parmi SEQ ID N010, SEQ ID N011, SEQ ID N012, SEQ ID N040, SEQ ID N041, et leurs séquences complémentaires, chacun desdits fragments étant notamment une sonde, pour obtenir une composition diagnostique, prophylactique ou thérapeutique, pour détecter un premier agent pathogène et/ou infectant, et un second agent pathogène et/ou infectant, associés à la polyarthrite rhumatoïde, ou une réactivation de l'un ou l'autre dudit premier agent et dudit second agent,
xx) l'utilisation d'une association comprenant un premier polypeptide codé de manière partielle ou totale par le premier fragment nucléotidique défini en ix), et un second polypeptide codé de manière partielle ou totale par le deuxième fragment nucléotidique défini en ix), pour obtenir une composition diagnostique, prophylactique ou thérapeutique, pour détecter un premier agent pathogène et/ou infectant, et un second agent pathogène et/ou infectant, associés à la polyarthrite rhumatoïde ; selon une utilisation avantageuse, cette association comprend un premier ligand, notamment anticorps, spécifique du premier polypeptide, et un second ligand, notamment anticorps, spécifique du second polypeptide, lesdits premier et second polypeptides étant définis ci-dessus,
xxi) un procédé d'obtention d'un premier agent pathogène et/ou infectant défini à l'un quelconque des points i) à vi) et/ou d'un second agent pathogène et/ou infectant défini à l'un quelconque des points x) à xii), associés à la polyarthrite rhumatoïde, caractérisé en ce qu'on cultive in vitro des cellules de ponctions de liquide synovial notamment choisies parmi les synoviocytes et les fibroblastes desquamés de liquide articulaire, de patients atteints de polyarthrite rhumatoïde,
xxii) un procédé d'obtention d'un premier agent pathogène et/ou infectant défini à l'un quelconque des points i) à vi) et/ou d'un second agent pathogène et/ou infectant défini à l'un quelconque des points x) à xii), associés à la polyarthrite rhumatoïde, caractérisé en ce qu'on cultive in vitro des cellules lymphocytaires B immortalisées par le virus d'Epstein-Barr, de patients atteints de polyarthrite rhumatoïde.
xxiii) un fragment nucléotidique, dont la séquence nucléotidique comprend une séquence choisie parmi SEQ ID N039, SEQ ID N042, SEQ ID N043, leurs séquences complémentaires, et leurs séquences équivalentes, notamment les séquences nucléotidiques présentant pour toute suite de 100 monomères contigus, au moins 50 %, et de préférence au moins 70 % d'homologie, avec une séquence choisie parmi SEQ ID N039, SEQ ID N042, SEQ ID N043, et leurs séquences complémentaires ;
xxiv) une amorce spécifique pour l'amplification par polymérisation d'un ARN ou d'un ADN d'un matériel viral défini à l'un quelconque des points i) ii) iii) iv) v)) vi), comprenant une séquence nucléotidique identique ou équivalente à au moins une partie de la séquence nucléotidique d'un fragment selon xxiii), notamment une séquence nucléotidique présentant, pour toute suite de 10 monomères contigus, au moins 70 % d'homologie avec au moins une partie dudit fragment ; en particulier cette amorce comprend une séquence nucléotidique choisie parmi SEQ ID N047, SEQ ID N048, SEQ ID N049, et leurs séquences complémentaires ;
xxv) une sonde susceptible de s'hybrider spécifiquement avec un ARN ou un ADN d'un matériel viral défini à l'un quelconque des points i) ii) iii) iv) v) vi) comprenant une séquence nucléotidique identique ou équivalente à au moins une partie de la séquence nucléotidique d'un fragment selon xxiii), notamment une séquence nucléotidique présentant, pour toute suite de 10 monomères contigus, au moins 70 % d'homologie avec au moins une partie dudit fragment ; en particulier cette sonde comprend une séquence nucléotidique choisie parmi SEQ ID N047, SEQ ID N048, SEQ ID N049, et leurs séquences complémentaires ;
xxvi) un fragment nucléotidique, dont la séquence nucléotidique comprend une séquence choisie parmi SEQ ID N040, SEQ ID N041, leurs séquences complémentaires, et leurs séquences équivalentes, notamment les séquences nucléotidiques présentant pour toute suite de 100 monomères contigus, au moins 70 %, et de préférence au moins 90 % d'homologie, avec une séquence choisie parmi SEQ ID N040, SEQ ID N041, et leurs séquences complémentaires ;
xxvii) une amorce spécifique pour l'amplification par polymérisation d'un ARN ou d'un ADN d'un agent pathogène et/ou infectant défini à l'un quelconque des points x) xi) xii), comprenant une séquence nucléotidique identique ou équivalente à au moins une partie de la séquence nucléotidique d'un fragment selon xxvi), notamment une séquence nucléotidique présentant, pour toute suite de 10 monomères contigus, au moins 90 % d'homologie avec au moins une partie dudit fragment ; en particulier cette amorce comprend une séquence nucléotidique choisie parmi SEQ ID N044, SEQ ID N045, SEQ ID N046, et leurs séquences complémentaires ;
xxviii) une sonde susceptible de s'hybrider spécifiquement avec un ARN ou un ADN d'un matériel viral défini à l'un quelconque des points x) xi) xii) , comprenant une séquence nucléotidique identique ou équivalente à au moins une partie de la séquence nucléotidique d'un fragment selon xxvi), notamment une séquence nucléotidique présentant, pour toute suite de 10 monomères contigus, au moins 90% d'homologie avec au moins une partie dudit fragment ; en particulier cette sonde comprend une séquence nucléotidique choisie parmi SEQ ID N044, SEQ ID N045, SEQ ID N046, et leurs séquences complémentaires.

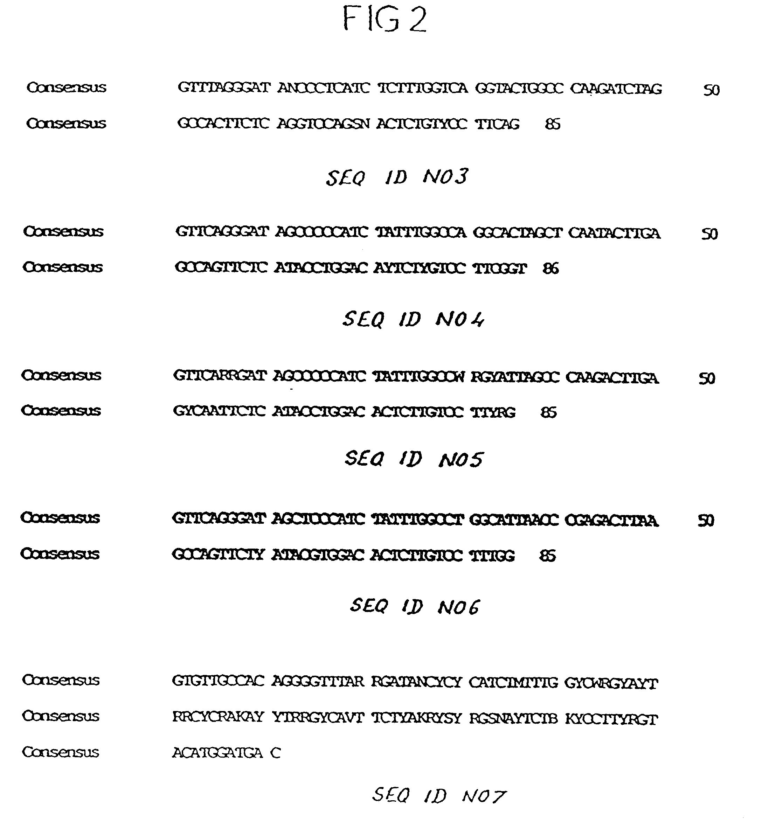

Avant de détailler l'invention, différents termes utilisés dans la description et les revendications sont à présent définis :
- par souche ou isolat, on entend toute fraction biologique infectante et/ou pathogène, contenant par exemple des virus et/ou des bactéries et/ou des parasites, et générant un pouvoir pathogène et/ou antigénique, hébergée par une culture ou un hôte vivant ; à titre d'exemple; une souche virale selon la définition précédente peut contenir un agent co-infectant, par exemple un protiste pathogène,
- le terme "MSRV" utilisé dans la présente description désigne tout agent pathogène et/ou infectant, associé à la SEP ou à la PR, notamment une espèce virale, les souches atténuées de ladite espèce virale, ou les particules défectives interférentes dérivées de cette espèce. Il est connu que les virus et particulièrement les virus contenant de l'ARN ont une variabilité, consécutive notamment à des taux relativement élevés de mutation spontanée **(9)**, dont il sera tenu compte ci-après pour définir la notion d'équivalence,
- par virus humain, on entend un virus susceptible d'infecter l'être humain,
- compte tenu de toutes les variations naturelles ou induites, pouvant être rencontrées dans la pratique de la présente invention, les objets de cette dernière, définis ci-dessus et dans les revendications, ont été exprimés en comprenant les équivalents ou dérivés des différents matériels biologiques définis ci-après, notamment des séquences homologues nucléotidiques ou peptidiques,
- le variant d'un virus ou d'un agent pathogène et/ou infectant selon l'invention, comprend au moins un antigène reconnu par au moins un anticorps dirigé contre au moins un antigène correspondant dudit virus et/ou dudit agent pathogène et/ou infectant, et/ou un génome dont toute partie est détectée par au moins une sonde d'hybridation, et/ou au moins une amorce d'amplification nucléotidique spécifique dudit virus et/ou agent pathogène et/ou infectant, comme par exemple celles ayant une séquence nucléotidique choisie parmi SEQ ID N013 à SEQ ID N038, et SEQ ID N044 à SEQ ID N049, et leurs séquences complémentaires, dans des conditions d'hybridation déterminées bien connues de l'homme de l'art,
- selon l'invention, un fragment nucléotidique ou un oligonucléotide ou un polynucléotide est un enchaînement de monomères, ou un biopolymère, caractérisé par la séquence informationnelle des acides nucléiques naturels, susceptibles de s'hybrider à tout autre fragment nucléotidique dans des conditions prédéterminées, l'enchaînement pouvant contenir des monomères de structures chimiques différentes et être obtenu à partir d'une molécule d'acide nucléique naturelle et/ou par recombinaison génétique et/ou par synthèse chimique,
- ainsi un monomère peut être un nucléotide naturel d'acide nucléique, dont les éléments constitutifs sont un sucre, un groupement phosphate et une base azotée; dans l'ARN le sucre est le ribose, dans l'ADN le sucre est le désoxy-2-ribose; selon qu'il s'agit de l'ADN ou l'ARN, la base azotée est choisie parmi l'adénine, la guanine, l'uracile, la cytosine, la thymine; ou le nucléotide peut être modifié dans l'un au moins des trois éléments constitutifs ; à titre d'exemple, la modification peut intervenir au niveau des bases, générant des bases modifiées telles que l'inosine, la méthyl-5-désoxycytidine, la désoxyuridine, la diméthylamino-5-désoxyuridine, la diamino-2,6-purine, la bromo-5-désoxyuridine et toute autre base modifiée favorisant l'hybridation; au niveau du sucre, la modification peut consister dans le remplacement d'au moins un désoxyribose par un polyamide **(10)**, et au niveau du groupement phosphate, la modification peut consister dans son remplacement par des esters, notamment choisis parmi les esters de diphosphate, d'alkyl et arylphosphonate et de phosphorothioate,
- par "séquence informationnelle", on entend toute suite ordonnée de monomères, dont la nature chimique et l'ordre dans un sens de référence, constituent ou non une information fonctionnelle de même qualité que celle des acides nucléiques naturels,
- par hybridation, on entend le processus au cours duquel, dans des conditions opératoires appropriées, deux fragments nucléotidiques, ayant des séquences suffisamment complémentaires, s'apparient pour former un brin multiple, notamment double ou triple, de préférence sous forme d'hélice,
- une sonde comprend un fragment nucléotidique synthétisé par voie chimique ou obtenu par digestion ou coupure enzymatique d'un fragment nucléotidique plus long, comprenant au moins six monomères, avantageusement de 10 à 100 monomères, de préférence 10 à 30 monomères, et possédant une spécificité d'hybridation dans des conditions déterminées ; de préférence, une sonde possédant moins de 10 monomères n'est pas utilisée seule, mais l'est en présence d'autres sondes de taille aussi courte ou non ; dans certaines conditions particulières, il peut être utile d'utiliser des sondes plus longues, par exemple de taille supérieure à 100 monomères ; une sonde peut notamment être utilisée à des fins de diagnostic et il s'agira par exemple de sondes de capture et/ou de détection,
- la sonde de capture peut être immobilisée sur un support solide par tout moyen approprié, c'est-à-dire directement ou indirectement, par exemple par covalence ou adsorption passive,
- la sonde de détection peut être marquée au moyen d'un marqueur choisi notamment parmi les isotopes radioactifs, des enzymes notamment choisis parmi la péroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine,
- les sondes utilisées à des fins de diagnostic de l'invention peuvent être mises en oeuvre dans toutes les techniques d'hybridation connues et notamment les techniques dites "DOT-BLOT" **(11)**, "SOUTHERN BLOT" **(12)**, "NORTHERN BLOT" qui est une technique identique à la technique "SOUTHERN BLOT" mais qui utilise de l'ARN comme cible, la technique SANDWICH **(13)**; avantageusement, on utilise la technique SANDWICH dans la présente invention, comprenant une sonde de capture spécifique et/ou une sonde de détection spécifique, étant entendu que la sonde de capture et la sonde de détection doivent présenter une séquence nucléotidique au moins partiellement différente,
- l'invention couvre également une sonde susceptible de s'hybrider in vivo ou in vitro sur l'ARN et/ou sur l'ADN, pour bloquer les phénomènes de réplication, notamment traduction et/ou transcription, et/ou pour dégrader ledit ADN et/ou ARN,
- une amorce est une sonde comprenant au moins six monomères, et avantageusement de 10 à 30 monomères, possédant une spécificité d'hybridation dans des conditions déterminées, pour l'initiation d'une polymérisation enzymatique par exemple dans une technique d'amplification telle que la PCR (Polymerase Chain Reaction), dans un procédé d'élongation, tel que le séquençage, dans une méthode de transcription inverse ou analogue,
- deux séquences nucléotidiques ou peptidiques sont dites équivalentes ou dérivées l'une par rapport à l'autre, ou par rapport à une séquence de référence, si fonctionnellement les biopolymères correspondants peuvent jouer sensiblement le même rôle, sans être identiques, vis-à-vis de l'application ou utilisation considérée, ou dans la technique dans laquelle elles interviennent ; sont notamment équivalentes deux séquences obtenues du fait de la variabilité naturelle, notamment mutation spontanée de l'espèce à partir de laquelle elles ont été identifiées, ou induite, ainsi que des séquences homologues, l'homologie étant définie ci-après,
- par variabilité, on entend toute modification, spontanée ou induite d'une séquence, notamment par substitution, et/ou insertion, et/ou délétion de nucléotides et/ou de fragments nucléotidiques, et/ou extension et/ou racourcissement de la séquence à l'une au moins des extrémités ; une variabilité non naturelle peut résulter des techniques de génie génétique utilisées, par exemple du choix des amorces de synthèse dégénérées ou non, retenues pour amplifier un acide nucléique; cette variabilité peut se traduire par des modifications de toute séquence de départ, considérée comme référence, et pouvant être exprimées par un degré d'homologie par rapport à ladite séquence de référence,
- l'homologie caractérise le degré d'identité de deux fragments nucléotidiques ou peptidiques comparés; elle se mesure par le pourcentage d'identité qui est notamment déterminé par comparaison directe de séquences nucléoditiques ou peptidiques, par rapport à des séquences nucléotidiques ou peptidiques de référence,
- ce pourcentage d'identité a été spécifiquement déterminé pour les fragments nucléotidiques relevant de la présente invention, homologues aux fragments identifiés par SEQ ID N01 à SEQ ID N09 et SEQ ID N039, SEQ ID N042, SEQ ID N043, (MSRV-1) d'une part, et ceux homologues aux fragments identifiés, par SEQ ID NO10 à SEQ ID N012, et SEQ ID N040 à SEQ ID N043 (MSRV-2), d'autre part, ainsi que pour les sondes et amorces homologues aux sondes et amorces identifiées par SEQ ID N016 à SEQ ID N026, SEQ ID N031 à SEQ ID N033, et SEQ ID N047 à SEQ ID N049, d'une part, et aux sondes et amorces identifiées par SEQ ID N013 à N015, SEQ ID N027 à SEQ ID N030, SEQ ID N034 à N037, et SEQ ID N044 à N046, d'autre part ; à titre d'exemple, le plus faible pourcentage d'identité observé entre les différents consensus généraux en acides nucléiques obtenus à partir de fragments d'ARN viral de MSRV-1, issu des lignées LM7PC et PLI-2 selon un protocole détaillé plus loin, est de 67% dans la région décrite à la figure 2,
- tout fragment nucléotidique est dit équivalent ou dérivé d'un fragment de référence, s'il présente une séquence nucléotidique équivalente à la séquence de référence ; selon la définition précédente, sont notamment équivalents à un fragment nucléotidique de référence :
   a) tout fragment susceptible de s'hybrider au moins partiellement avec le complément du fragment de référence
   b) tout fragment dont l'alignement avec le fragment de référence conduit à mettre en évidence des bases contigues identiques, en nombre plus important qu'avec tout autre fragment provenant d'un autre groupe taxonomique
   c) tout fragment résultant ou pouvant résulter de la variabilité naturelle de l'espèce, à partir de laquelle il est obtenu
   d) tout fragment pouvant résulter des techniques de génie génétique appliquées au fragment de référence
   e) tout fragment, comportant au moins huit nucléotides contigus, codant un peptide homologue ou identique au peptide codé par le fragment de référence,
   f) tout fragment différent du fragment de référence, par insertion, délétion, substitution d'au moins un monomère, extension, ou raccourcissement à l'une au moins de ses extrémités; par exemple tout fragment correspondant au fragment de référence flanqué à l'une au moins de ses extrémités par une séquence nucléotidique ne codant pas pour un polypeptide,
- par polypeptide, on entend notamment tout peptide d'au moins deux acides aminés, notamment oligopeptide, protéine, extrait, séparé, ou substantiellement isolé ou synthétisé, par l'intervention de la main de l'homme, notamment ceux obtenus par synthèse chimique, ou par expression dans un organisme recombinant,
- par polypeptide codé de manière partielle par un fragment nucléotidique, on entend un polypeptide présentant au moins 3 acides aminés codés par au moins 9 monomères contigus compris dans ledit fragment nucléotidique,
- un acide aminé est dit analogue à un autre acide aminé, lorsque leur caractéristiques physico-chimiques respectives, telles que polarité, hydrophobicité, et/ou basicité, et/ou acidité, et/ou neutralité, sont sensiblement les mêmes; ainsi, une leucine est analogue à une isoleucine.
- tout polypeptide est dit équivalent ou dérivé d'un polypeptide de référence, si les polypeptides comparés ont sensiblement les mêmes propriétés, et notamment les mêmes propriétés antigéniques, immunologiques, enzymologiques et/ou de reconnaissance moléculaire ; est notamment équivalent à un polypeptide de référence :
   a) tout polypeptide possédant une séquence dont au moins un acide aminé a été substitué par un acide aminé analogue,
   b) tout polypeptide ayant une séquence peptidique équivalente, obtenue par variation naturelle ou induite dudit polypeptide de référence, et/ou du fragment nucléotidique codant pour ledit polypeptide,
   c) un mimotope dudit polypeptide de référence,
   d) tout polypeptide dans la séquence duquel un ou plusieurs acides aminés de la série L sont remplacés par un acide aminé de la série D, et vice versa,
   e) tout polypeptide dont l'une au moins des liaisons CO-NH, et avantageusement toutes les liaisons CO-NH, de la chaîne peptidique du peptide parent correspondant, (ne comportant pas de liaison NH-CO dans sa chaîne peptidique), est (sont) remplacée(s) par une (des) liaison(s) NH-CO,
   f) tout polypeptide dont l'une au moins des liaisons CO-NH, et avantageusement toutes les liaisons CO-NH, de la chaîne peptidique du peptide parent correspondant, (ne comportant pas de liaison NH-CO dans sa chaîne peptidique), est (sont) remplacée(s) par une (des) liaison(s) NH-CO, la chiralité de chaque résidu aminoacyle, qu'il soit impliqué ou non dans une ou plusieurs liaisons CO-NH sus-mentionnées, étant soit conservée, soit inversée par rapport aux résidus aminoacyles correspondants constituant ledit peptide parent, ces composés du type peptidique étant encore désignés immunorétroïdes,
   g) tout polypeptide dans la séquence duquel on a introduit une modification des chaînes latérales des acides aminés, telle que par exemple une acétylation des fonctions amines, une carboxylation des fonctions thiol, une estérification des fonctions carboxyliques,
   h) tout polypeptide dans la séquence duquel une ou des liaisons peptidiques ont été modifiées comme par exemple les liaisons carba, rétro, inverso, rétro-inverso, réduites, et méthylène-oxy.
   i) tout polypeptide dont au moins un antigène est reconnu par un antigène du polypeptide de référence,
- le pourcentage d'identité caractérisant l'homologie de deux fragments peptidiques comparés est selon la présente invention d'au moins 50% et de préférence au moins 70%.

Etant donné qu'un virus possédant une activité enzymatique transcriptase inverse peut être génétiquement caractérisé aussi bien sous forme d'ARN que d'ADN, il sera fait mention aussi bien de l'ADN que de l'ARN viral pour caractériser les séquences relatives à un virus possédant une telle activité transcriptase inverse (MSRV-1).

Etant donné que l'agent pathogène et/ou infectant (MSRV)-2 a été détecté tant en ADN qu'en ARN dans les cellules infectées, il peut également être caractérisé sous forme d'ADN ou ARN.

Les expressions d'ordre utilisées dans la présente description et les revendications, telles que "première séquence nucléotidique" ne sont pas retenues pour exprimer un ordre particulier, mais pour définir plus clairement l'invention.

L'invention sera mieux comprise à la lecture de la description détaillée qui va suivre faite en référence aux figures annexées dans lesquelles:
- la figure 1 représente la séquence de type MSRV-2A obtenue à partir des cultures LM7 selon le protocole de Shih **(8)** ; cette séquence est identifiée sous la référence SEQ ID N010,
- la figure 2 représente des consensus généraux en acides nucléiques des séquences MSRV-lB amplifiées par la technique PCR dans la région "pol", à partir d'ADN viral issu des lignées LM7PC et PLI-2, identifiés sous les références SEQ ID N03, SEQ ID N04, SEQ ID N05, et SEQ ID N06, et le consensus commun avec amorces d'amplification portant la référence SEQ ID N07,
- la figure 3 représente l'arbre phylogénétique des séquences de type MSRV-lB obtenues par PCR dans la région "pol" définie par Shih **(8)**,
- la figure 4 donne la définition d'une trame de lecture fonctionnelle pour chaque famille de type MSRV-lB/"PCR pol", lesdites familles A à D étant définies respectivement par les sequences nucléotidiques SEQ ID N03, SEQ ID N04, SEQ ID N05, et SEQ ID N06, décrites à la figure 2,
- la figure 5 donne un exemple de consensus des séquences de MSRV-2B, identifié par SEQ ID N011,
- la figure 6 représente la séquence nucléotidique du clone PSJ17 (SEQ ID N09),
- la figure 7 représente la séquence nucléotidique SEQ ID N08, du clone dénommé M003-P004,
- la figure 8 représente la séquence nucléotidique SEQ ID N02 du clone F11-1; la partie repérée entre deux flèches dans la région de l'amorce correspond à une variabilité imposée par le choix de l'amorce ayant servi au clonage de F11-1; sur cette même figure, la traduction en acides aminés est représentée,
- la figure 9 représente la séquence nucléotidique SEQ ID N01, et une trame fonctionnelle de lecture possible en acides aminés de SEQ ID N01; sur cette séquence, les séquences consensus des transcriptases inverses rétrovirales sont soulignées,
- la figure 10 représente la séquence nucléotidique SEQ ID N012 du clone dénommé MSRV2EL1,
- la figure 11 décomposée en trois planches successives 11/23 à 13/23 représente la traduction en acides aminés de SEQ ID N012, incluant l'amorce SEQ ID N013, selon 6 trames de lecture possibles,
- la figure 12 présente un alignement de la séquence MSRV2-A (SEQ ID N010) sur la séquence MSRV2-EL1 (SEQ ID N012); sur cette même représentation, la région d'hybridation de l'amorce identifiée sous la référence SEQ ID N013, (hormis la queue de clonage) est encadrée; celle de l'amorce identifiée sous la référence SEQ ID N014, est signalée entre des crochets,

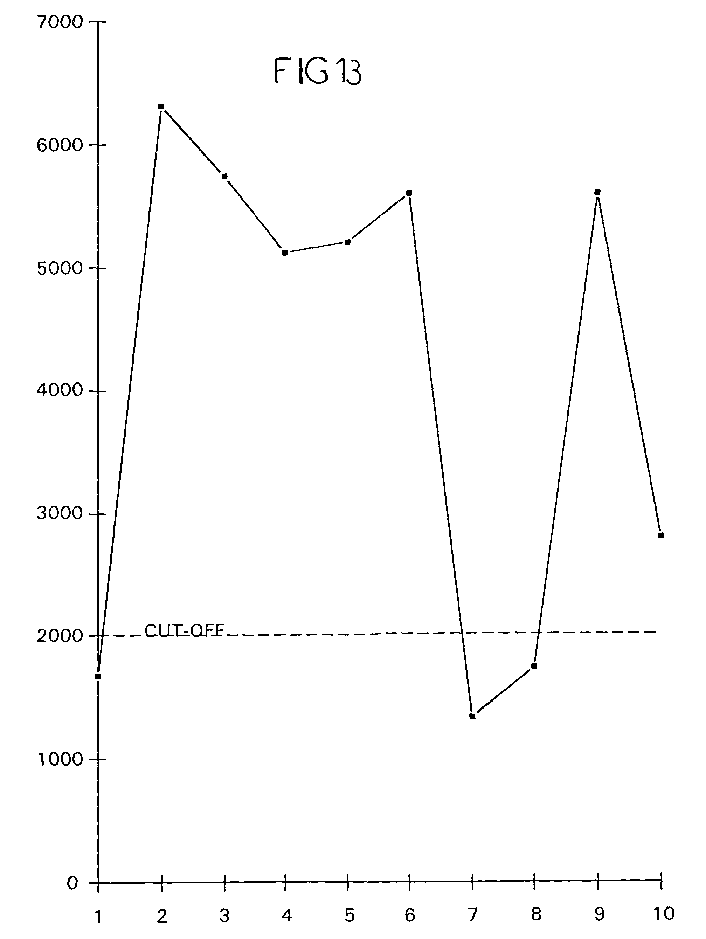

- la figure 13 représente la mesure de l'activité transcriptase inverse avec les conditions définies dans l'exemple 7, dans les fractions d'un gradient de saccharose où a été réalisée une sédimentation à l'équilibre d'agents infectants et/ou pathogènes présents dans le surnageant de cultures de cellules de liquides articulaires de polyarthrite rhumatoïde, ainsi que cela est décrit dans l'exemple 7 ; la courbe représente les variations de l'activité transcriptase inverse dans les différentes fractions du gradient ; cette activité est mesurée en DPM (désintégrations par minute) sur l'axe des ordonnées ; l'axe des abscisses représente les fractions collectées sur le gradient par ordre de densité croissante (fractions 1 à 10),
- la figure 14, divisée en figures 14A, 14B et 14C, montre, selon la figure 14A, la séquence du clone "MSRV-1polPR" identifiée par SEQ ID N039, obtenu sur échantillon de PR dans les conditions définies dans l'exemple 9, selon les figures 14B et 14C, l'alignement de la séquence de ce clone avec les SEQ ID N01 et SEQ ID N06, respectivement, correspondant à des séquences du rétrovirus MSRV-1 obtenues dans des échantillons provenant de SEP,
- la figure 15, divisée en figures 15A, 15B et 15C, montre selon la figure 15A, la séquence du clone "MSRV2sPR" identifiée par SEQ ID N040, obtenu sur échantillon de PR dans les conditions définies dans l'exemple 9, selon les figures 15B et 15C, l'alignement de la séquence de ce clone avec les SEQ ID N010 et SEQ ID N012, respectivement, correspondant à des séquences de l'agent infectant MSRV-2 obtenues dans des échantillons provenant de SEP,
- la figure 16 montre la séquence du clone MSRV2cPR identifiée par SEQ ID N041, obtenu à partir de cellules d'un liquide articulaire de PR, dans les conditions définies dans l'exemple 10,
- la figure 17 montre la séquence du clone MSRV2cPR identifiée par SEQ ID N041, obtenu à partir de cellules d'un liquide articulaire de PR, et l'alignement de la partie terminale (654-705) de la séquence de ce clone avec la SEQ ID N010, correspondant à une séquence de l'agent infectant MSRV-2 obtenue dans des échantillons provenant de SEP,
- la figure 18 montre la séquence du clone MSRV2cPR identifiée par SEQ ID N041, obtenu à partir de cellules d'un liquide articulaire de PR, et l'alignement de la séquence de ce clone avec la SEQ ID N012, correspondant à une séquence de l'agent infectant MSRV-2 obtenue dans des échantillons provenant de SEP,
- la figure 19 montre la séquence du clone MSRV1nPR identifiée par SEQ ID N042, obtenu à partir d'un liquide articulaire de PR, dans les conditions définies dans l'exemple 10,
- la figure 20 montre la séquence du clone MSRV1nPR identifiée par SEQ ID N043, obtenu à partir de cellules d'un liquide articulaire de PR, et l'alignement de la séquence de ce clone avec la SEQ ID N01, correspondant à une séquence du rétrovirus MSRV-1 obtenue à partir d'échantillons provenant de SEP.

### EXEMPLE 1: OBTENTION DE CLONES MSRV-2 DENOMMES MSRV-2A, PAR AMPLIFICATION DES REGIONS CONSERVEES DES GENES D'ADN-POLYMERASES ARN-DEPENDANTES, SUR UNE PREPARATION D'AGENT INFECTANT PURIFIE A PARTIR DE CULTURE DE CELLULES DE LA LIGNEE LM7

L'approche moléculaire a consisté à utiliser une technique PCR **(8)** qui permet d'amplifier une région relativement conservée du gène *pol* des rétrovirus exogènes et endogènes, mais aussi des virus codant pour une enzyme à activité transcriptase inverse (RT) tels que notamment le virus de l'hépatite B et, implicitement, de tout gène d'ADN polymérase ARN dépendante ou d'enzyme, présentant des homologies de séquences suffisantes dans les régions définies par les amorces d'amplification utilisées. Cette technique PCR a été utilisée sur les acides nucléiques extraits d'une préparation d'agent infectant purifiée, obtenue selon le protocole **(14)** à partir des surnageants de la culture LM7 d'origine **(7)** gardés congelés à -80°C depuis lors. Les fractions contenant le pic d'activité RT de type LM7 sont reprises dans un volume d'un tampon contenant du thiocyanate de guanidine **(15)** et sont stockées à -80°C jusqu'à extraction des acides nucléiques selon la technique décrite par P.Chomzynski **(15)**.

Préalablement à la réaction PCR, l'ARN de l'échantillon a été transcrit en ADN complémentaire (ADNc) avec des amorces dites "random" (hexanucléotides en mélange) à l'aide du Kit "cDNA synthesis system plus" (Amersham), selon les instructions du fabricant et en se basant sur une valeur approximée à un log près de la quantité d'ARN présente dans l'échantillon.

L'ADN obtenu après amplification PCR de l'ADNc, a été inséré dans un plasmide à l'aide du kit TA Cloning® (British Biotechnology). Les 2 µl de solution d'ADN ont été mélangés avec 5 µl d'eau distillée stérile, 1 µl d'un tampon de ligation 10 fois concentré "10X LIGATION BUFFER", 2 µl de "pCR^{TM} VECTOR" (25 ng/ml) et 1 µl de "TA DNA LIGASE". Ce mélange a été incubé une nuit à 12°C. Les étapes suivantes ont été réalisées conformément au instructions du kit TA Cloning®. A la fin de la procédure, les colonies blanches de bactéries recombinantes ont été repiquées pour être cultivées et permettre l'extraction des plasmides incorporés selon la procédure dite de "miniprep" **(16)**. La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du "TA cloning kit". La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer", modèle 373 A Applied Biosystems selon les instructions du fabricant.

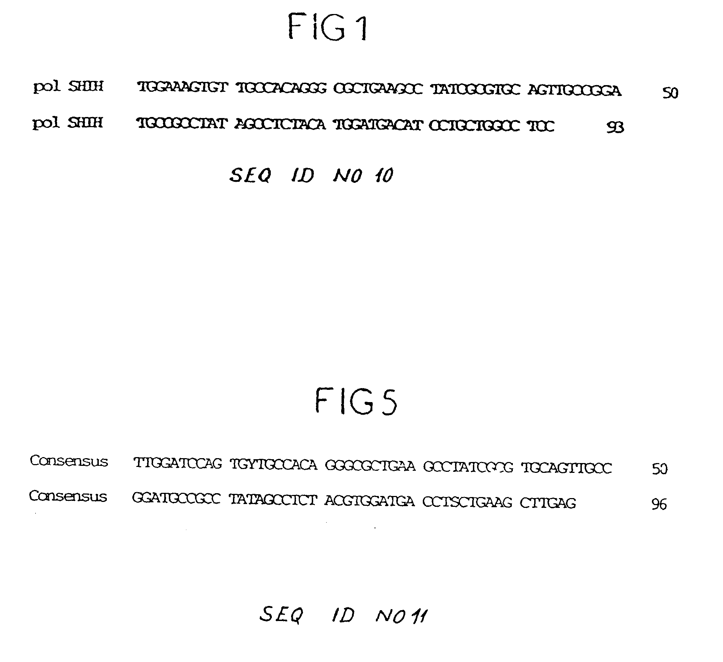

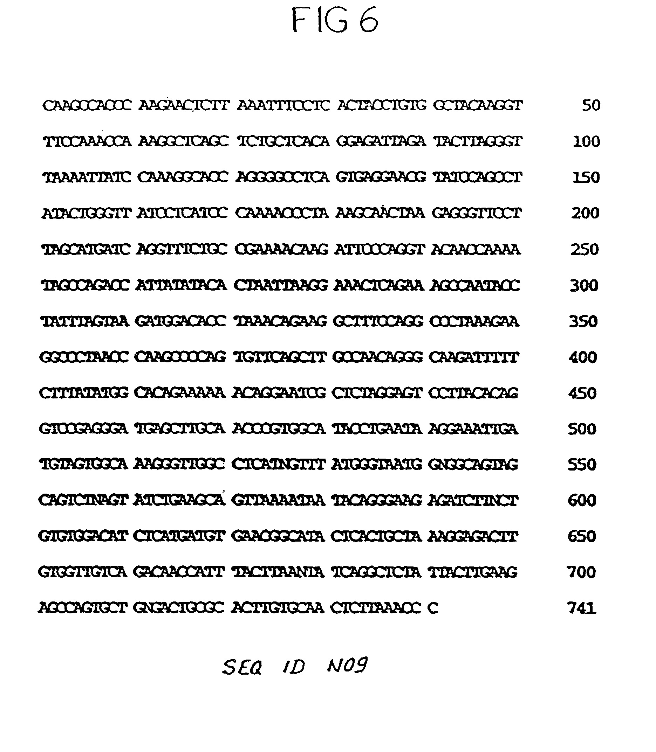

Les séquences obtenues ont ensuite été analysées à l'aide des logiciels Mac Vector® et Geneworks® sur banque de données informatiques Genebank®, pour les séquences nucléiques, et Swiss Prot®, pour les séquences en acides aminés déduites des trames de lecture mises en évidence dans les séquences nucléiques. L'analyse des séquences obtenues à partir de l'échantillon viral provenant des surnageants LM7 décongelés, et purifié au pic d'activité transcriptase inverse sur gradient de saccharose, a mis en évidence une population majoritaire de clones (environ 42% des clones), relativement à la représentativité individuelle des autres séquences (toujours inférieure à 5%, voire 10% pour un petit nombre), et présentant des homologies partielles avec des rétrovirus connus dans la région "pol" attendue. Ce clone est dénommé MSRV2-A et identifié par SEQ ID N010 (Cf. Fig. 1). La région amplifiée entre les amorces PCR est homologue à la séquence correspondante MSRV2-B identifiée par SEQ ID N011 (Cf. Fig. 5), décrite dans l'exemple 2. Les différences observées dans les séquences situées au niveau des amorces PCR s'expliquent par l'utilisation d'amorces dégénérées en mélange, utilisées dans des conditions techniques différentes. L'interrogation de la banque de données Genebank® actualisée à ce jour n'a pas permis de mettre en évidence une séquence identique ou présentant des homologies significatives.

Cette séquence est présentée dans la figure 1. La séquence présentée à la figure 1 possède un cadre de lecture ouvert en phase avec les deux amorces PCR retrouvées aux extrémités, mais elle est plus courte que l'ensemble des séquences rétrovirales connues dans la région attendue entre ces amorces. Une délétion de 45 paires de bases (15 acides aminés) y est observée, relativement aux séquences rétrovirales correspondantes **(8)**, à la suite de la séquence de l'amorce amont alors que les séquences précédant l'amorce aval sont présentes. Cependant la trame de lecture est ouverte et ininterrompue sur toute la séquence incluant les amorces et la séquence en acides aminés déduite présente une homologie significative avec la région correspondante des rétrovirus connus. Dans la séquence interne aux amorces PCR, les acides aminés Glu, Arg, Gln, Pro et Asp, normalement assez bien conservés dans cette région *pol* des rétrovirus et des virus avec activité transcriptase inverse connus **(8)** sont retrouvés conservés aux bonnes positions dans la trame de lecture de la séquence originale.

Enfin, étant donné que cette séquence est suffisamment divergente des séquences rétrovirales déjà décrites dans les banques de données, on peut avancer qu'il s'agit d'une séquence appartenant à un nouvel agent infectant et/ou pathogène, dénommé MSRV-2A. Cet agent s'apparente à priori, d'après l'analyse des séquences obtenues, à un rétrovirus, mais, étant donnée la technique utilisée pour obtenir cette séquence, il peut aussi s'agir d'un virus à ADN dont le génome code pour une enzyme qui possède accessoirement une activité transcriptase inverse comme c'est le cas, par exemple, pour le virus de l'hépatite B, HBV **(8)**. De plus, le caractère aléatoire des amorces dégénérées utilisées pour cette technique d'amplification PCR peut très bien avoir permis, du fait d'homologies de séquences imprévues ou de sites conservés dans le gène d'une enzyme apparentée, l'amplification d'un acide nucléique provenant d'un agent pathogène et/ou co-infectant procaryote ou eucaryote (protiste).

### EXEMPLE 2: OBTENTION DE CLONES DENOMMES MSRV-1B ET MSRV-2B, DEFINISSANT RESPECTIVEMENT UN RETROVIRUS MSRV-1 ET UN AGENT CO-INFECTANT MSRV2, PAR AMPLIFICATION PCR "NICHEE" DES REGIONS POL CONSERVEES DES RETROVIRUS, SUR DES PREPARATIONS DE VIRIONS ISSUES DES LIGNEES LM7PC ET PLI-2

Une technique PCR dérivée de la technique publiée par Shih **(8)** a été utilisée. Cette technique permet, par traitement de tous les composants du milieu réactionnel par la DNase, d'éliminer toute trace d'ADN contaminant. Elle permet parallèlement, par l'utilisation d'amorces différentes mais chevauchantes dans deux séries successives de cycles d'amplifications PCR, d'augmenter les chances d'amplifier un ADNc synthétisé à partir d'une quantité d'ARN faible au départ et encore réduite dans l'échantillon par l'action parasite de la DNAse sur l'ARN. En effet, la DNase est utilisée dans des conditions d'activité en excès qui permettent d'éliminer toute trace d'ADN contaminant avant inactivation de cette enzyme restant dans l'échantillon par chauffage à 85°C pendant 10 minutes. Cette variante de la technique PCR décrite par Shih **(8)**, a été utilisée sur un ADNc synthétisé à partir des acides nucléiques de fractions de particules infectantes purifiées sur gradient de saccharose selon la technique décrite par H. Perron **(14)** à partir de l'isolat "POL-2" (ECACC n°V92072202) produit par la lignée PLI-2 (ECACC n°92072201) d'une part, et à partir de l'isolat MS7PG (ECACC n°V93010816) produit par la lignée LM7PC (ECACC n°93010817) d'autre part. Ces cultures ont été obtenues selon les méthodes ayant fait l'objet des demandes de brevet publiées sous les n° WO 93/20188 et WO 93/20189.

Après clonage avec le TA Cloning Kit® des produits amplifiés par cette technique et analyse de la séquence à l'aide du séquençeur automatique selon ce qui est décrit dans l'exemple 1, les séquences ont été analysées à l'aide du logiciel Geneworks®, sur la dernière version disponible de la banque de données Genebank®.

Les séquences clonées et séquencées à partir de ces échantillons correspondent notamment à deux types de séquences: un premier type de séquence, retrouvé dans la majorité des clones (55% des clones issus des isolats POL-2 des culture PLI-2, et, 67% des clones issus des isolats MS7PG des cultures LM7PC) qui correspond à une famille de séquences "pol" proches mais différentes du rétrovirus endogène humain dénommé ERV-9 ou HSERV-9, et un second type de séquence qui correspond à des séquences très fortement homologues à la séquence attribuée à un agent infectant et/ou pathogène dénommé précédemment MSRV-2.

Le premier type de séquences représentant la majorité des clones est constituée de séquences dont la variabilité permet de définir quatre sous-familles de séquences. Ces sous-familles sont suffisamment proches entre elles pour qu'on puisse les considérer comme des quasi-espèces provenant d'un même rétrovirus, tel que cela est bien connu pour le rétrovirus HIV-1 **(17)**, ou comme le résultat de l'interférence avec plusieurs provirus endogènes co-régulés dans les cellules productrices. Ces éléments endogènes plus ou moins défectifs sont sensibles aux mêmes signaux de régulation générés éventuellement par un provirus réplicatif, puisqu'ils appartiennent à la même famille de rétrovirus endogènes **(18)**. Cette nouvelle famille de rétrovirus endogènes ou, alternativement, cette nouvelle espèce rétrovirale dont on a obtenu en culture la génération de quasi-espèces, et qui contient un consensus des séquences décrites ci-dessous est dénommée MSRV-1B.

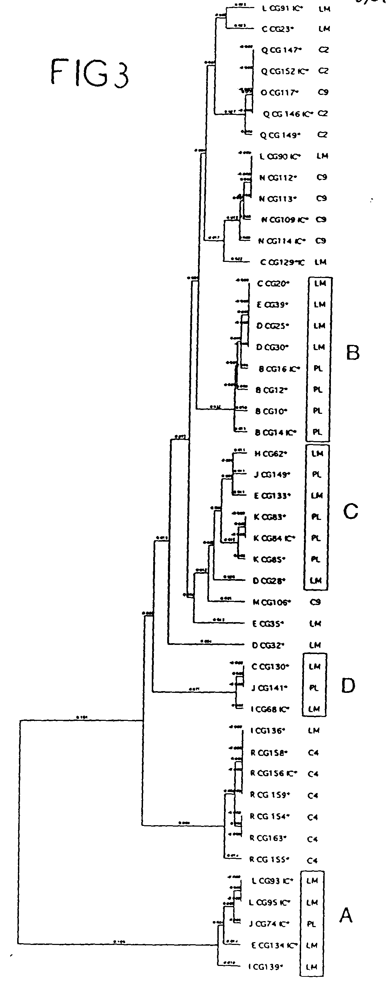

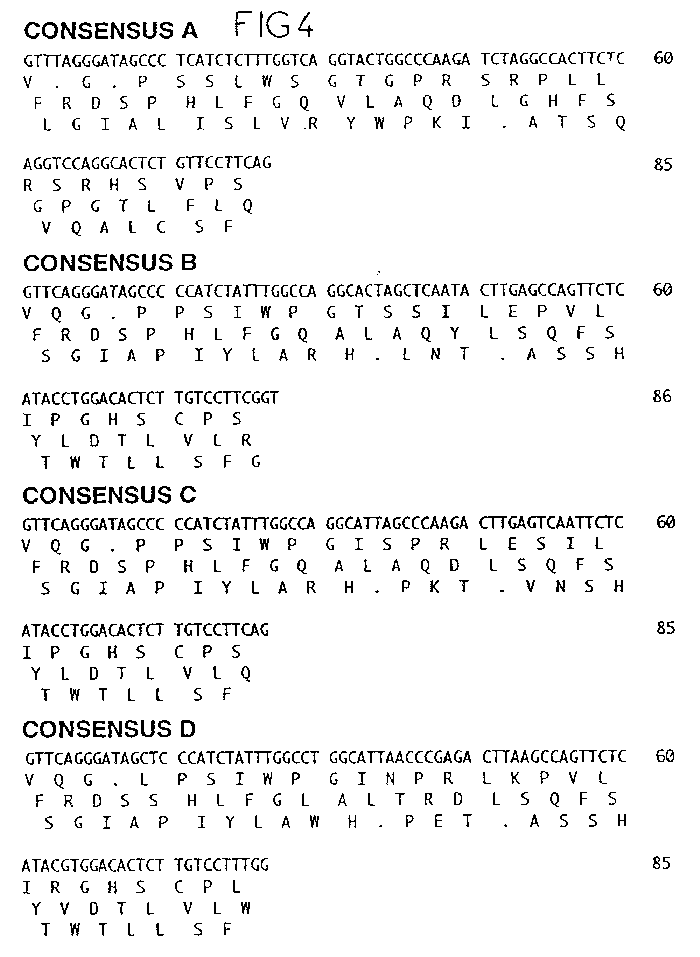

Dans la figure 2 sont présentées les consensus généraux des séquences des différents clones MSRV-1B séquencés lors de cette expérience, ces séquences étant respectivament identifiées par SEQ ID N03, SEQ ID N04, SEQ ID N05, et SEQ ID N06. Ces séquences présentent une homologie en acides nucléiques allant de 70% à 88% avec la séquence HSERV9 référencée X57147 et M37638 dans la base de données Genebank®. L'arbre phylogénétique de ces séquences est présenté dans la figure 3. Dans cette figure, les sous-familles A, B, C, D représentent les séquences qui ont été retrouvées de manière prépondérante dans des expériences similaires répétées ultérieurement, dans les échantillons d'ARN pur de virions purifiés à partir des isolats MS7PG et POL-2. A partir de ces familles de séquences, quatre séquences nucléiques "consensus" représentatives de différentes quasi-espèces d'un rétrovirus MSRV-1B éventuellement exogène, ou de différentes sous-familles d'un rétrovirus endogène MSRV-1B, ont été définies. Ces consensus représentatifs sont présentés dans la figure 4, avec la traduction en acides aminés. Une trame de lecture fonctionnelle existe pour chaque sous-famille de ces séquences MSRV-1B, et l'on peut voir que la trame de lecture ouverte fonctionnelle correspond à chaque fois à la séquence en acides aminés venant en deuxième ligne sous la séquence en acide nucléiques. Le consensus général de la séquence MSRV-1B, identifié par SEQ ID N07 et obtenu par cette technique PCR dans la région "pol" est présenté dans la figure 2.

Le deuxième type de séquence représentant la majorité des clones séquencés est représenté par la séquence MSRV-2B présentée dans la figure 5 et identifiée par SEQ ID N011. La région amplifiée entre les amorces PCR est homologue à une base près à la séquence MSRV2-A (SEQ ID NO10 selon Fig.1) interne aux amorces PCR décrite dans l'Exemple 1. Les différences observées dans les séquences correspondant aux amorces PCR s'expliquent par l'utilisation d'amorces dégénérées en mélange utilisées dans des conditions techniques différentes.

Les séquences MSRV-2A (SEQ ID N010) et MSRV-2B (SEQ ID N011) sont à l'évidence homologues, voire identiques, issues du même organisme et suffisamment divergentes des séquences rétrovirales déjà décrites dans les banques de données pour qu'on puisse avancer qu'il s'agit d'une région de séquence appartenant à un nouvel agent infectant, dénommé MSRV-2. Cet agent infectant s'apparenterait à priori, d'après l'analyse des premières séquences obtenues, à un rétrovirus, mais, étant donnée la technique utilisée pour obtenir cette séquence, il pourrait aussi s'agir d'un virus à ADN dont le génome code pour une enzyme qui possède accessoirement une activité transcriptase inverse comme c'est le cas, par exemple, pour le virus de l'hépatite B, HBV **(8)**. De plus, le caractère aléatoire des amorces dégénérées utilisées pour cette technique d'amplification PCR peut très bien avoir permis, du fait d'homologies de séquences imprévues ou de sites conservés dans le gène d'une enzyme apparentée, l'amplification d'un acide nucléique provenant d'un agent pathogène et/ou co-infectant prokaryote ou eukaryote (protiste).

### EXEMPLE 3: OBTENTION D'UN CLONE PSJ17, DEFINISSANT UN RETROVIRUS MSRV-1, PAR REACTION DE TRANSCRIPTASE INVERSE ENDOGENE SUR UNE PREPARATION DE VIRION ISSUE DE LA LIGNEE PLI-2.

Cette approche vise à obtenir des séquences d'ADN rétrotranscrites à partir de l'ARN supposé rétroviral dans l'isolat, en utilisant l'activité transcriptase inverse présente dans ce même isolat. Cette activité transcriptase inverse ne peut théoriquement fonctionner qu'en présence d'un ARN rétroviral, lié à un ARNt amorce ou hybridé à des brins courts d'ADN déjà rétro-transcrits dans les particules rétrovirales **(19)**. Ainsi l'obtention de séquences rétrovirales spécifiques dans un matériel contaminé par des acides nucléiques cellulaires, a été optimisée selon ces auteurs grâce à l'amplification enzymatique spécifique des portions d'ARN viraux par une activité transcriptase inverse virale. Les auteurs ont pour cela, déterminé les conditions physico-chimiques particulières dans lesquelles cette activité enzymatique de transcription inverse sur des ARN contenus dans des virions pouvait être effective in vitro. Ces conditions correspondent à la description technique des protocoles présentés ci-dessous (réaction de RT endogène, purification, clonage et séquençage).

L'approche moléculaire a consisté à utiliser une préparation de virion concentré, mais non purifié, obtenu à partir des surnageants de culture de la lignée PLI-2 préparés selon la méthode suivante : les surnageants de culture sont collectés deux fois par semaine, pré-centrifugés à 10 000 trs/min pendant 30 minutes pour éliminer les débris cellulaires, et ensuite, congelés à -80°C ou utilisés tels que pour les étapes suivantes. Les surnageants frais ou décongelés sont centrifugés sur un coussin de PBS-glycérol 30% à 100 000g (ou 30 000 trs/min dans un rotor LKB-HITACHI de type 45 T) pendant 2 h à 4°C. Après élimination du surnageant, le culot sédimenté est repris dans un petit volume de PBS et constitue la fraction de virion concentré, mais non purifié. Cet échantillon viral concentré mais non purifié a été utilisé pour effectuer une réaction dite de transcription inverse endogène, telle que décrite ci-après : un volume de 200 µl de virion purifié selon le protocole décrit ci-dessus et contenant une activité transcriptase inverse d'environ 1-5 millions de dpm est décongelé à 37°C jusqu'à apparition d'une phase liquide, puis placé sur de la glace. Un tampon 5 fois concentré a été préparé avec les composants suivants: Tris-HCl pH 8,2, 500 mM; NaCl 75 mM; MgCl2 25 mM; DTT 75 mM et NP 40 0,10 %. 100 µl de tampon 5X + 25 µl d'une solution de dATP 100 mM + 25 µl d'une solution de dTTP 100 mM + 25 µl d'une solution de dGTP 100 mM + 25 µl d'une solution de dCTP 100 mM + 100 µl d'eau distillée stérile + 200 µl de la suspension de virions (activité T.I. de 5 millions de DPM) dans le PBS ont été mélangés et incubés à 42°C pendant 3 heures. Après cette incubation le mélange réactionnel est directement ajouté à un mélange tamponné phénol/chloroforme/alcool isoamylique (Sigma ref. P 3803); la phase aqueuse est collectée et un volume d'eau distillée stérile est ajouté à la phase organique pour ré-extraire le matériel nucléique résiduel. Les phases aqueuses collectées sont regroupées et les acides nucléiques contenus sont précipités par addition d'acétate de sodium 3M, pH 5,2 au 1/10 de volume + 2 volumes d'éthanol + 1 µl de glycogène (Boehringer-Mannheim ref. 901 393) et mise à -20°C de l'échantillon pendant 4 h ou la nuit à +4°C. Le précipité obtenu après centrifugation est ensuite lavé avec de l'éthanol à 70% et resuspendu dans 60 ml d'eau distillée. Les produits de cette réaction ont ensuite été purifiés, clonés et séquencés selon les protocole décrit ci-après: des ADN bouts francs avec des adénines non-appariées aux extrémités ont été générés: une réaction de "remplissage" a d'abord été effectuée: 25 µl de la solution d'ADN précédemment purifiée ont été mélangés avec 2 µl d'une solution 2,5 mM contenant, en quantité equimolaire, dATP + dGTP + dTTP + dCTP / 1 µl d'ADN polymérase T4 (Boehringer-Mannheim ref. 1004 786) / 5 µl de 10X "incubation buffer for restriction enzyme" (Boehringer-Mannheim ref. 1417 975) / 1 µl d'une solution à 1% de sérum-albumine bovine / 16 µl d'eau distillée stérile. Ce mélange a été incubé 20 minutes à 11°C. 50 µl de tampon TE et 1 µl de glycogène (Boehringer-Mannheim ref. 901 393) y ont été ajoutés avant extraction des acides nucléiques avec du phénol/chloroforme/alcool isoamylique (Sigma ref. P 3803), et précipitation à l'acétate de sodium comme décrit précédemment. L'ADN précipité après centrifugation est resuspendu dans 10 µl de tampon 10 mM Tris pH 7,5. Puis, 5 µl de cette suspension ont été mélangés avec 20µl de tampon Taq 5X, 20 µl de 5mM dATP, 1µl (5U) de Taq ADN polymérase (Amplitaq^{TM}) et 54 µl d'eau distillée stérile. Ce mélange est incubé 2 h à 75°C avec un film d'huile à la surface de la solution. L'ADN en suspension dans la solution aqueuse prélevée en dessous du film d'huile après incubation est précipité comme décrit précédemment et resuspendu dans 2 µl d'eau distillée stérile. L'ADN obtenu a été inséré dans un plamide à l'aide du kit TA Cloning^{TM}. Les 2 µl de solution d'ADN ont été mélangés avec 5 µl d'eau distillée stérile, 1µl d'un tampon de ligation 10 fois concentré "10X LIGATION BUFFER", 2 µl de "pCR^{TM} VECTOR" (25 ng/ml) et 1 µl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" **(16)**. La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer, modèle 373 A" Applied Biosystems, selon les instructions du fabricant.

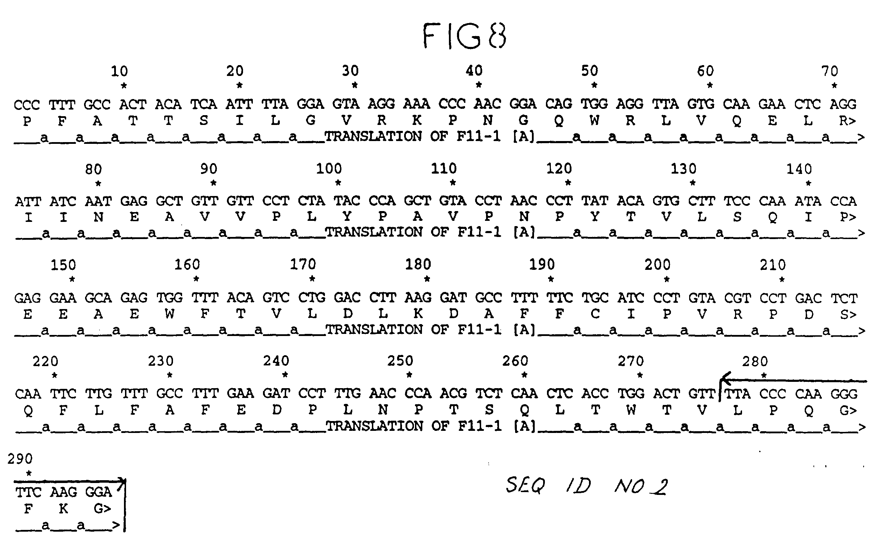

L'analyse discriminante sur les banques de données informatiques des séquences clonées à partir des fragments d'ADN présents dans le mélange réactionnel a permis de mettre en évidence une séquence de type rétroviral. Le clone correspondant PSJ17 a été entièrement séquencé et la séquence obtenue, présentée dans la figure 8 et identifiée apr SEQ ID N09, a été analysée à l'aide du logiciel "Geneworks®" sur les banques de données actualisées "Genebank®". L'analyse des banques de données n'a pas permis de trouver de séquence identique déjà décrite. Seule une homologie partielle a pu être retrouvée avec certains éléments rétroviraux connus. L'homologie relative la plus intéressante concerne un rétrovirus endogène dénommé ERV-9, ou HSERV-9, selon les références **(20)**.

### EXEMPLE 4: AMPLIFICATION PCR DE LA SEQUENCE NUCLEIQUE CONTENUE ENTRE LA REGION 5' DEFINIE PAR LE CLONE "POL MSRV-1B" ET LA REGION 3' DEFINIE PAR LE CLONE PSJ17.

Cinq oligonucléotides, M001, M002-A, M003-BCD, P004 et P005, ont été définis pour amplifier de l'ARN provenant de virions purifiés POL-2. Des réactions de contrôle ont été effectuées de manière à contrôler la présence de contaminants (réaction sur de l'eau). L'amplification consiste en une étape de RT-PCR selon le protocole décrit dans l'Exemple 2, suivie d'une PCR "nichée" selon le protocole PCR décrit dans le document EP-A-0569272. Dans le premier cycle RT-PCR, les amorces M001 et P004 ou P005 sont utilisées. Dans le deuxième cycle PCR, les amorces M002-A ou M003-BCD, et l'amorce POO4 sont utilisées. Les amorces sont positionnées comme suit:

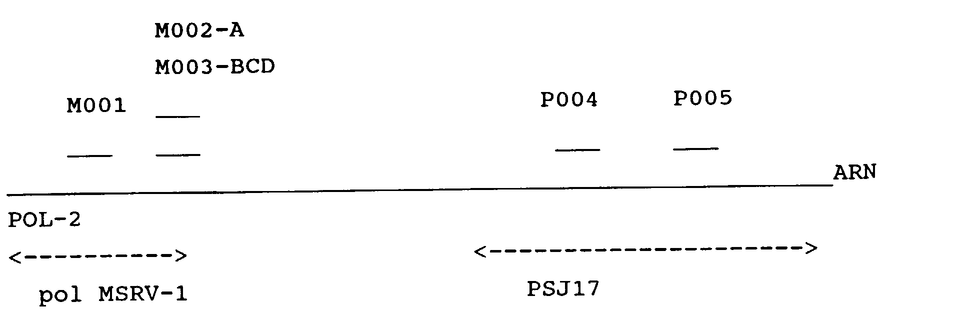

Leur composition est:
amorce M001: GGTCITITCCCICAIGG (SEQ ID NO20) amorce M002-A: TTAGGGATAGCCCTCATCTCT (SEQ ID N021) amorce M003-BCD: TCAGGGATAGCCCCCATCTAT(SEQ ID NO22) amorce P004: AACCCTTTGCCACTACATCAATTT (SEQ ID NO23) amorce P005:

GCGTAAGGACTCCTAGAGCTATT (SEQ ID N024)

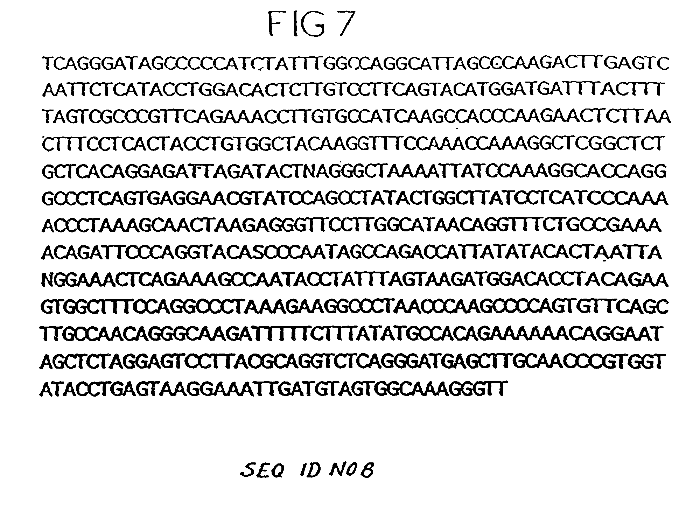

Le produit d'amplification "nichée" obtenu et dénommé M003-P004 est présenté dans la figure 7, et correspond à la séquence SEQ ID N08.

### EXEMPLE 5: AMPLIFICATION ET CLONAGE D'UNE PORTION DU GENOME RETROVIRAL MSRV-1 A L'AIDE D'UNE SEQUENCE DEJA IDENTIFIEE, DANS UN ECHANTILLON DE VIRUS PURIFIE AU PIC D'ACTIVITE TRANSCRIPTASE INVERSE

Une technique PCR dérivée de la technique publiée par Frohman **(21)** a été utilisée. La technique dérivée permet, à l'aide d'une amorce spécifique en 3' du génome à amplifier, d'élonguer la séquence vers la région 5' du génome à analyser. Cette variante technique est décrite dans la documentation de la firme "Clontech Laboratories Inc., (Palo-Alto California, USA) fournie avec son produit "5'-AmpliFINDER^{TM} RACE Kit", qui a été utilisé sur une fraction de virion purifié comme décrit précédemment.

Les amorces 3' spécifiques utilisées dans le protocole du kit pour la synthèse du cDNA et l'amplification PCR sont respectivement complémentaires aux séquences MSRV-1 suivantes:

cDNA :      TCATCCATGTACCGAAGG      (SEQ ID N025)

amplification : ATGGGGTTCCCAAGTTCCCT (SEQ ID
NO26)

Les produits issus de la PCR ont été purifiés après purification sur gel d'agarose selon les méthodes conventionnelles **(16)**, puis resuspendus dans 10 ml d'eau distillée. Une des propriétés de la polymérase Taq consistant à ajouter une adénine à l'extémité 3' de chacun des deux brins d'ADN, l'ADN obtenu a été directement inséré dans un plamide à l'aide du kit TA Cloning^{TM} (British Biotechnology). Les 2 µl de solution d'ADN ont été mélangés avec 5 µl d'eau distillée stérile, 1 µl d'un tampon de ligation 10 fois concentré "10X LIGATION BUFFER", 2 µl de "pCR^{TM} VECTOR" (25 ng/ml) et 1 µl de "TA DNA LIGASE". Ce mélange a été incubé une nuit à 12°C. Les étapes suivantes ont été réalisées conformément aux instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" **(16)**. La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "automatic sequencer, modèle 373 A" Applied Biosystems selon les instructions du fabricant.

Cette technique a d'abord été appliquée à deux fractions de virion purifié comme décrit ci-après, sur saccharose à partir de l'isolat "POL-2" produit par la lignée PLI-2 d'une part, et à partir de l'isolat MS7PG produit par la lignée LM7PC d'autre part: les surnageants de cultures sont collectés deux fois par semaine, pré-centrifugés à 10 000 trs/min pendant 30 minutes pour éliminer les débris cellulaires, et ensuite, congelés à _80°C ou utilisés tels quels pour les étapes suivantes. Les surnageants frais ou décongelés sont centrifugés sur un coussin de PBS-glycérol 30% à 100 000g (ou 30 000 trs/min dans un rotor LKB-HITACHI de type 45 T) pendant 2 h à 4°C. Après élimination du surnageant, le culot sédimenté est repris dans un petit volume de PBS et constitue la fraction de virions concentrés, mais non purifiés. Le virus concentré est ensuite déposé sur un gradient de sucrose dans un tampon PBS stérile (15 à 50% poids/poids) et ultracentrifugé à 35 000 trs/min (100 000g) pendant 12 h à +4°C dans un rotor à godets. 10 fractions sont recueillies et 20 µl sont prélevés dans chaque fraction après homogénéisation pour y doser l'activité transcriptase inverse selon la technique décrite par H. Perron **(7)**. Les fractions contenant le pic d'activité RT "de type LM7" sont ensuite diluées dans du tampon PBS stérile et ultracentrifugées une heure à 35 000 trs/min (100 000g) pour sédimenter les particules virales. Le culot de virion purifié ainsi obtenu est alors repris dans un petit volume d'un tampon adéquat pour l'extraction d'ARN. La réaction de synthèse de cDNA mentionnée plus haut est réalisée sur cet ARN extrait de virion extracellulaire purifié. L'amplification PCR selon la technique citée plus-haut a permis d'obtenir le clone F1-11 dont la séquence identifiée par SEQ ID N02, est présentée dans la figure 8.

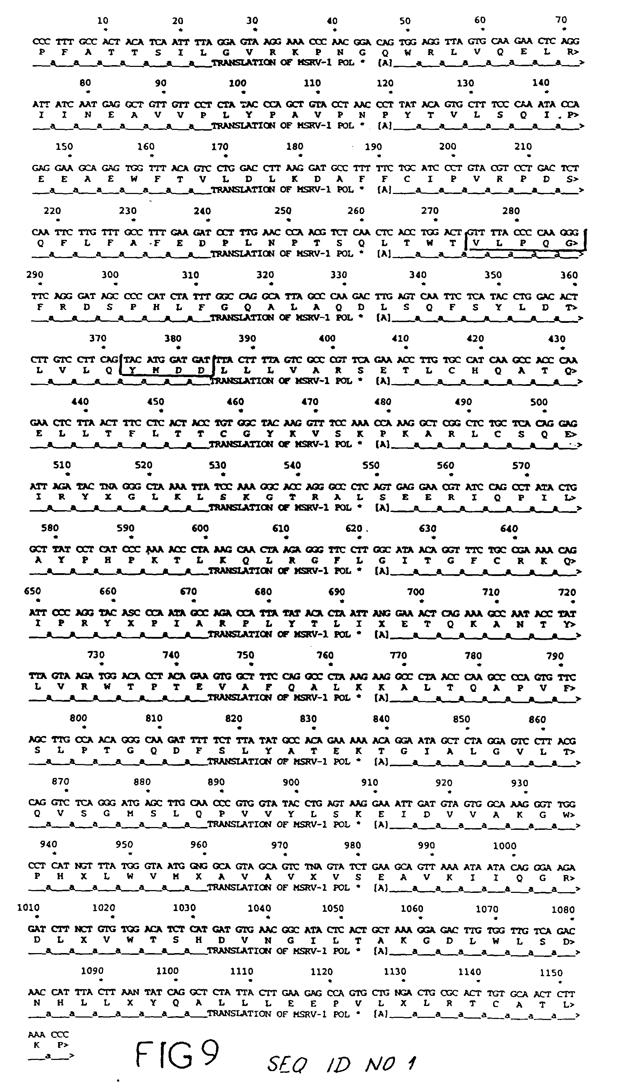

Ce clone permet de définir, avec les différents clones préalablement séquencés, une région représentative du gène "pol" du rétrovirus MSRV-1, telle que présentée dans la figure 9. Cette séquence dénommée SEQ ID N01 est reconstituée à partir de différents clones se recouvrant à leurs extrémités, en corrigeant les artéfacts liés aux amorces et aux techniques d'amplification ou de clonage, qui interromperaient artificiellement la trame de lecture de l'ensemble.

Dans la figure 9, la trame de lecture potentielle avec sa traduction en acides aminés est présentée en dessous de la séquence en acides nucléiques.

### EXEMPLE 6: CAPTURE, AMPLIFICATION ET CLONAGE D'UNE PORTION DU GENOME MSRV-2 A L'AIDE D'UNE SEQUENCE DEJA IDENTIFIEE, DANS UNE CULTURE INFECTEE PAR MSRV-2

Les surnageants d'une culture cellulaire exprimant une activité transcriptase inverse "de type LM7" similaire à celle décrite par H. Perron **(7)** ont été collectés régulièrement pendant plusieurs semaines et conservés congelés à -80°C après addition de 10% de glycérol. L'ensemble des surnageants a ensuite été décongelé afin de concentrer les particules infectantes par ultracentrifugation et de les purifier par centrifugation à l'équilibre sur gradient de saccharose; l'activité transcriptase inverse a ensuite été mesurée dans les différentes fractions collectées sur le gradient selon la méthodologie décrite par H. Perron **(14)**.

Les différentes fractions représentant le pic d'activité transcriptase inverse ont été regroupées afin d'en extraire les acides nucléiques selon un protocole destiné à la purification d'ARN **(15)**, mais les acides nucléiques extraits n'ont pas été traités par la DNase. Une amplification PCR dérivée de la technique décrite par Shih **(8)** a été effectuée directement sur cet échantillon d'acides nucléiques non traité par la DNase, selon un procédé d'amplification d'ARN tel que décrit dans le document EP-A-0 569 272, dans un volume total de 100 µl, contenant 200 ng d'ARN, 1µl d'ARN Guard, 33 µmoles de chaque mélange d'amorces (MOP) décrits par Shih **(8)**, et identiques à ceux utilisés pour la PCR (ADN) directe ; 0,25 mM de chaque dNTP, 10 µl de tampon 10X, 2,5 u d'enzyme *Taq* et 0,4 µl d'enzyme RT (RT-AMV; 10u) sont aussi ajoutés aux échantillons. Les cycles d'amplification sont réalisés comme suit: dénaturation de l'ARN 65°C/10 minutes, synthèse de l'ADNc 50°C/8 minutes, puis les cycles sont identiques à ceux de la PCR décrite par Shih **(8)**. Des réactions de contrôle ont été effectuées de manière à contrôler l'absence de contaminants (réaction sur de l'eau). Les produits ont été analysés sur gel d'acrylamide 10%.

Les échantillons amplifiés par RT-PCR ont ensuite été clonés et séquencés selon les techniques décrites dans l'exemple 1.

La majorité des clones séquencés à partir du produit de RT-PCR correspond à la séquence MSRV-2A et à son équivalente MSRV-2B précédemment décrites dans les exemples 1 et 2.

Par ailleurs, après élimination des séquences artéfactuelles, il s'avère que les autres clones séquencés correspondent à des séquences de type MSRV-1 telles que décrites dans les exemples 1 et 2.

Après la vérification des séquences présentes dans ce matériel nucléique provenant de ces fractions purifiées contenant des particules infectantes dont au moins une partie est associée à une activité transcriptase inverse, le matériel nucléique restant a été utilisé pour effectuer une capture spécifique des acides nucléiques portant la séquence MSRV2 préalablement identifiée et décrite dans les exemples 1 et 2.

Dans une étape préalable, le matériel génétique portant la séquence MSRV2 a été amplifié par une technique PCR monodirectionnelle de 50 cycles à l'aide d'une seule amorce. Cette amorce est couplée à une molécule de biotine à son extrémité 3', permet l'amplification monodirectionnelle de 3' vers 5' et correspond à la séquence suivante identifiée sous SEQ ID N038 :

5' TAAAGATCTAGAATTCGGCTATAGGCGGCATCCGGCAACT 3'

Ensuite, la capture a été effectuée en solution avec des billes magnétiques couplées à l'avidine (Dynabeads®) selon les instructions du fabricant (Dynal) et, après une série de lavages à température ambiante permettant d'éliminer les acides nucléiques non couplés à une biotine, une PCR a été effectuée directement sur ces billes lavées avec une amorce spécifique en 3' et une amorce en 5' apportée par une solution d'oligonucléotide de 10 bases (10 mer) avec une séquence aléatoire.

L'amorce d'amplification spécifique orientée de 3' vers 5' correspond à la séquence identifiée par SEQ ID N013:

5' GCATCCGGCAACTGCACG 3'

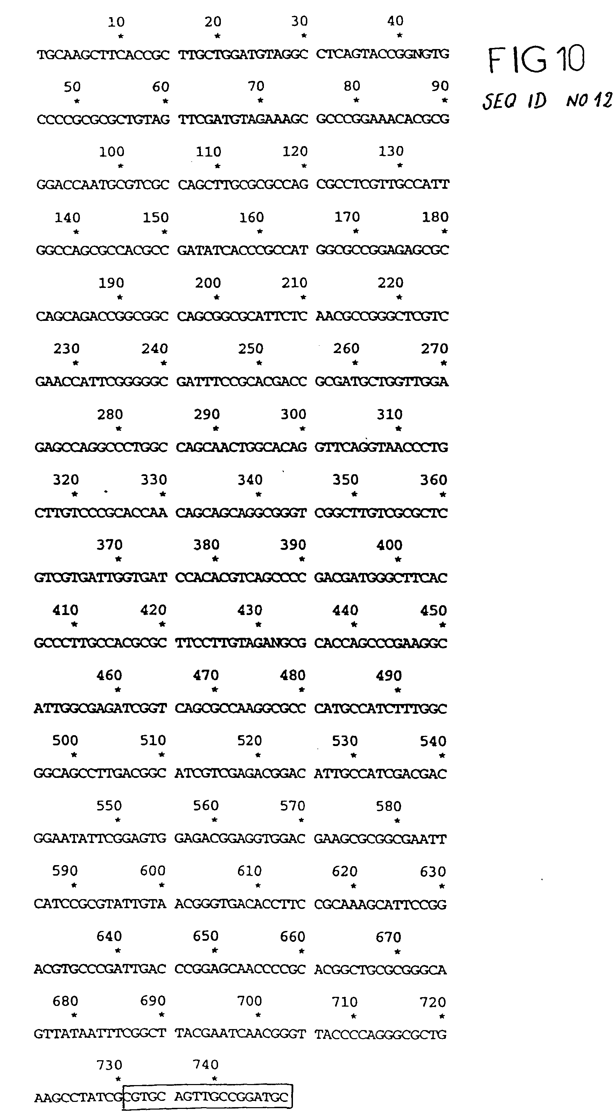

La PCR effectuée à 35°C sur 40 cycles avec ces amorces a permis d'amplifier le matériel génétique spécifiquement biotinilé par la première étape de PCR et capturé sur les billes Dynabeads®. Après clonage avec le kit "TA cloning" de l'ADN amplifié par cette seconde étape de PCR et séquençage des clones recombinants, selon les techniques décrites dans l'Exemple 1, une séquence de 748 paires de bases a été obtenue. Cette séquence d'acides nucléiques SEQ ID N012 est présentée dans la figure 10. Cette séquence élonguée sera dénommée ensuite MSRV-2EL1.

La séquence inverse complémentaire de l'amorce SEQ ID N013 est présente à l'extrémité 3' et est encadrée sur la figure 10. En amont de cette amorce, on retrouve la séquence déjà identifiée dans les clones MSRV-2A et MSRV-2B.

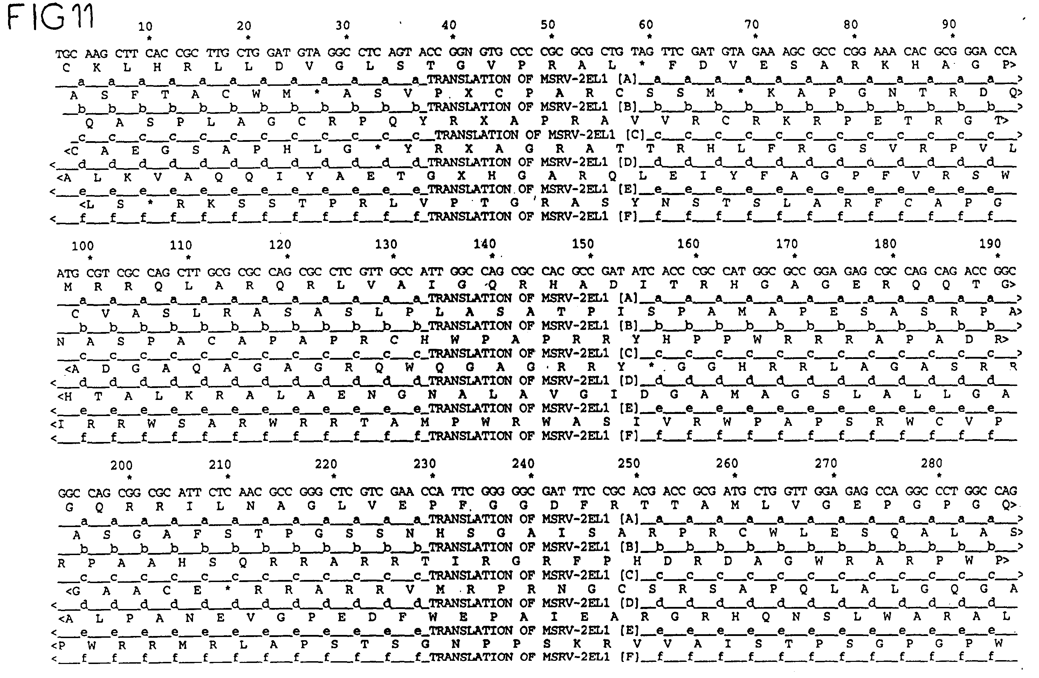

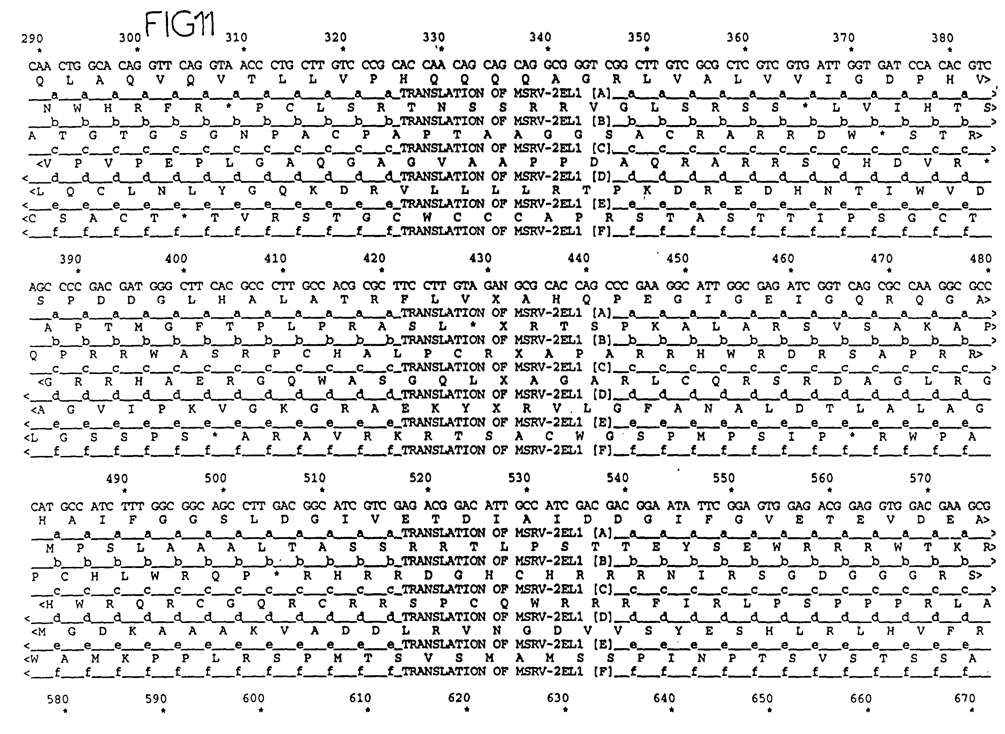

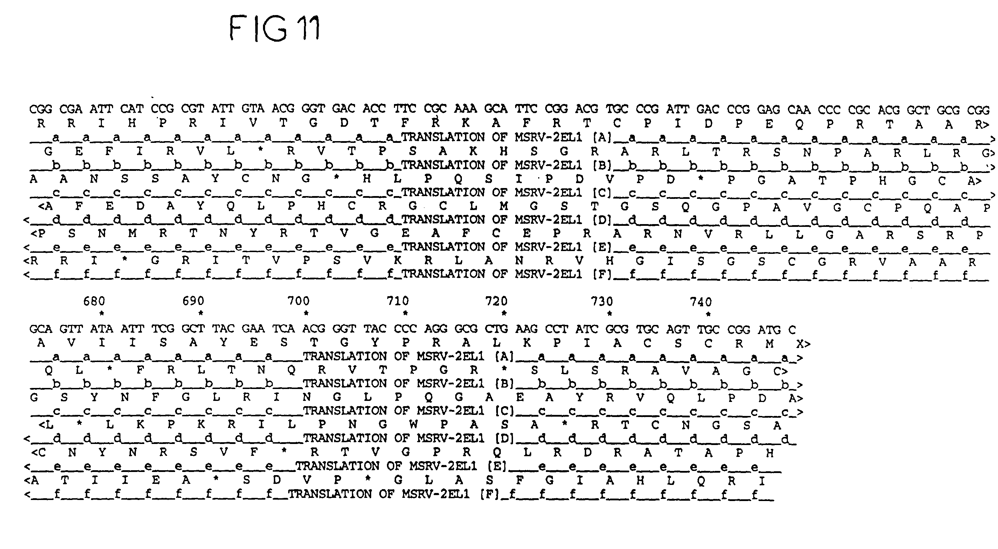

La traduction en acide aminés de cette séquence selon les 6 trames de lectures possibles est présentée dans la figure 11.

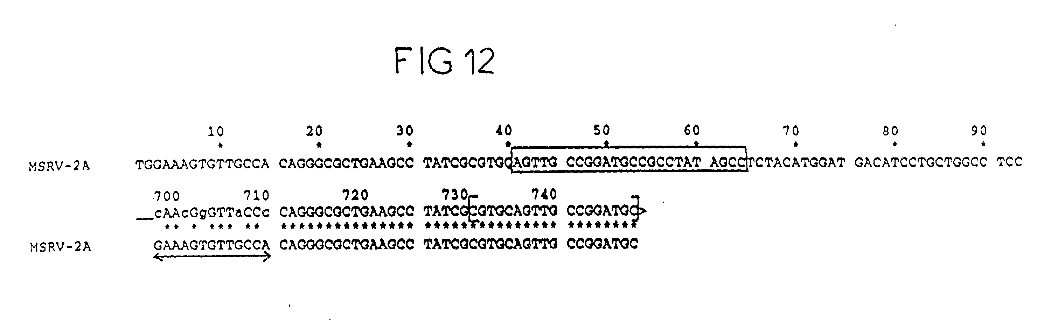

Un alignement de la séquence MSRV2-A (SEQ ID N010) sur la séquence MSRV-2EL1 (SEQ ID N012) est présenté dans la figure 12. On note que la séquence MSRV-2A est rigoureusement identique à la séquence élonguée, hormis quelques différences dans la région correspondant aux amorces dégénérées utilisées pour obtenir MSRV-2A. Cette région est soulignée dans cette figure ; par ailleurs, la région d'hybridation de l'amorce SEQ ID N013, (hormis la queue de clonage) est encadrée, celle de l'amorce SEQ ID N014 est présentée entre crochets. La séquence réelle du génome MSRV-2 dans cette région est vraisemblablement celle de MSRV-2EL1, où elle n'a pas été imposée par des amorces hybridées à basse stringence comme c'est le cas pour MSRV-2A (et MSRV-2B de même).

La séquence MSRV-2EL1 correspond donc à une nouvelle région séquencée du génome MSRV-2. Ceci a été vérifié à l'aide de nouvelles amorces PCR définies dans MSRV-2EL1 et MSRV-2A qui ont permis une amplification spécifique sur les acides nucléiques utilisés pour le clonage décrit dans cet exemple.

Le résultat d'interrogation de la banque de donnée Genebank®, actualisée en août 1994, avec la séquence MSRV-2EL1 ne montre aucune homologie significative avec des séquences génétiques connues à ce jour. Cependant, l'interrogation des traductions possibles en acides aminés selon les 6 trames de lecture potentielles de cette séquence MSRV-2EL1, montre des homologies partielles avec des séquences bactériennes, virales ou cellulaires.

L'absence d'amplification PCR avec des amorces spécifiques sur de l'ADN humain normal montre qu'il ne s'agit pas d'une séquence d'origine cellulaire. MSRV-2 est donc un agent infectant exogène à l'homme. Cependant, la nature dégénérée des mélanges d'amorces, utilisées selon des variantes de la technique décrite par Shih **(8)**, qui ont permis l'identification des premiers éléments de séquence dénommés MSRV-2A et MSRV-2B, peut avoir permis l'amplification imprévue d'un génome n'appartenant pas à un rétrovirus, ni même à un gène codant pour une ADN-polymérase ARN-dépendante.

### EXEMPLE 7 : CULTURE DE CELLULES DE LIQUIDES ARTICULAIRES DE PATIENTS ATTEINTS DE POLYARTHRITE RHUMATOÏDE (PR) ET DETECTION D'UNE ACTIVITE TRANSCRIPTASE SIMILAIRE A CELLE QUE PRODUIT LA CULTURE LM7.

Du liquide articulaire (LA) a été prélevé stérilement dans une articulation du genou présentant une atteinte inflammatoire, chez une patiente atteinte de polyarthrite rhumatoïde. Ce liquide a été centrifugé à +4°C, à 1800 trs/min pendant 10 minutes. Une fois le surnageant prélevé et aliquoté à -80°C, le culot cellulaire a été repris dans du milieu de culture RPMI dont la composition est la suivante: milieu RPMI 1640 additionné de pénicilline (200 000 U/L), de streptomycine (200 mg/L), de L-glutamine (6 mM/l), de pyruvate (1%), d'acides aminés non-essentiels (1%), éventuellement d'un anticorps polyclonal anti-interféron bêta humain (10 U/ml), et de 20% de sérum de veau foetal décomplémenté par incubation à 56°C pendant 30 minutes. Ces cellules en suspension ont été transférées dans un petit flacon de culture (25 cm³) et le volume complété à 5 ml avec le milieu sus-décrit dans lequel les cellules ont été maintenues en culture in vitro dans une étuve à 5% de CO₂ à 37°C.

Après 4 jours, le milieu de culture a été remplacé par du milieu frais de même composition et le milieu prélevé, centrifugé à 1800 trs/min pendant 10 minutes. Le surnageant est aliquoté et stocké à -80°C ; le culot constitué de cellules qui n'avaient pas adhéré au flacon de culture est repris dans du milieu de culture et transféré dans un autre flacon. Ces cellules seront maintenues en culture comme des cellules en suspension.

Les milieux de culture sont toujours changés au moins deux fois par semaine.

Après trois semaines de culture, le flacon dans lequel a été effectué le premier passage de cellules du liquide articulaire pathologique, contient des cellules adhérentes de type macrophagique et d'autres, de type fibroblastique, qui présentent uhe prolifération par "plages" de mitoses. Ces plages de prolifération ont progressivement recouvert la surface inférieure du flacon de culture et, au stade de confluence, ces cellules fibroblastiques ont été décollées par grattage et réparties dans deux flacons de 25 cm³ avec 5 ml de milieu de culture. Ces cellules adhérentes ont été ainsi passées et dédoublées tant que le potentiel de prolifération le permettait. En effet, après le sixième passage, soit environ deux mois de culture, les cellules fibroblastiques ont cessé de proliférer et les flacons ont été conservés jusqu'à dégénerescence des cellules.

Dans les flacons qui ont servi à passer les cellules en suspension, principalement des cellules de type lymphoïde, des cellules adhérentes de type macrophagique ou de type fibroblastique ont parfois adhéré tardivement. Les cellules lymphoïdes en suspension ont dégénéré sans donner lieu à une prolifération et n'ont pas été maintenues en culture après trois semaines.

L'ensemble des surnageants de culture de tous ces flacons a été stocké aliquoté à -80°C.

La recherche d'un éventuel agent rétroviral produit par les cellules de ce liquide articulaire pathologique d'une patiente atteinte de PR a d'abord consisté à rechercher une activité transcriptase inverse selon différentes conditions physico-chimiques permettant une détection non sélective d'une telle enzyme codée par un rétrovirus non connu à l'avance, ainsi que cela fut fait par H. Perron et coll., pour la recherche d'une activité transcriptase inverse dans des surnageants de culture de cellules du système nerveux de sclérose en plaques **(7)**. Les conditions de détection d'un signal spécifique d'une activité transcriptase inverse dans les surnageants de culture de cellules du LA de ce cas de polyarthrite rhumatoïde (PR) se sont alors révélées très proches de celles qui constituent l'optimum pour la détection du rétrovirus produit par la culture LM7 provenant d'un cas de sclérose en plaques (SEP). On a donc cherché à vérifier si cette activité pouvait être détectée dans les mêmes conditions que pour le rétrovirus MSRV1/LM7 isolé dans la SEP, après réalisation d'une sédimentation à l'équilibre sur gradient de saccharose, d'un concentrat de particules provenant des surnageants de cultures du LA de PR.

Une série de surnageants de culture des cellules adhérentes du LA de ce cas de PR a été décongelée afin de disposer d'un volume total d'au moins 100 ml. Ces surnageants ont été mélangés, pré-centrifugés afin d'éliminer les débris cellulaires, ultracentrifugés afin de sédimenter d'éventuelles particules rétrovirales et le culot ainsi sédimenté a été déposé sur un gradient de saccharose qui a été centrifugé à 100 000 g afin d'obtenir une séparation des éléments présents dans le culot d'ultracentrifugation et de doser, après recueil de fractions d'un volume égal et de densité de saccharose croissante, l'activité transcriptase inverse dans chacune de ces fractions. L'ensemble de ces opérations a été effectué dans les conditions décrites par H. Perron **(14)**.

Le résultat du dosage d'une activité transcriptase inverse avec les conditions optimisées pour la souche LM7, dans les fraction du gradient "PR" est présenté dans la figure 13. On peut voir qu'une activité transcriptase inverse significative (supérieure au cut-off de 2000 DPM) est retrouvée dans les fractions 2 à 5, ainsi que dans la fraction 9. L'activité retrouvée dans les fractions les moins denses est mal focalisée ce qui est probablement dû à un excès de matériel déposé sur le gradient (tube de 5 ml). Le pic secondaire dans la fraction plus dense (n°9) est vraisemblement constitué de virions plus denses ayant perdu leur enveloppe (capsides) ainsi que cela a déjà été observé dans les gradients réalisés avec des virions produits par la lignée LM7 **(14)**.

A ce stade des travaux effectués sur les cultures de cellules de LA provenant de PR, il semble que celles-ci produisent des particules possédant une transcriptase inverse du même type que celle qui est détectée dans les virions produits par les cellules LM7 de leptoméninges de SEP.

### EXEMPLE 8: CULTURE DE CELLULES LYMPHOBLASTOÏDES B DE PATIENTS ATTEINTS DE POLYARTHRITE RHUMATOÏDE (PR).

Selon un protocole bien connu de l'homme de métier, des lignées lymphoblastoïdes, immortalisées par adjonction in vitro de la souche B95 du virus d'Epstein-Barr (EBV) ou par la souche autologue hébergée par les lymphocytes B du patient, ont été établies à partir des lymphocytes B du sang périphérique de patients atteints de PR.

Brièvement, les cellules mononuclées du sang ont été séparées par centrifugation sur gradient de Ficoll-Hypaque® (Flow/ICN), lavées par centrifugation dans du RPMI 1640, puis reprises dans un milieu contenant du RPMI 1640 additionné de pénicilline (200 000 U/L), de streptomycine (200 mg/L), de L-glutamine (2 mM/l), de pyruvate (1%), d'acides aminés non-essentiels (1%), de tampon HEPES (1%) et de 20% de sérum de veau foetal décomplémenté par incubation à 56°C pendant 30 minutes. Ces cellules en suspension sont transférées dans un petit flacon de culture (25 cm³) et le volume est complèté à 5 ml avec le milieu sus-décrit dans lequel les cellules seront maintenues en culture in vitro dans une étuve à 5% de CO₂ à 37°C.

Les milieux sont renouvelés par moitié deux fois par semaine: après décantation pendant au moins 2 h des flacons placés verticalement, la moitié du milieu surnageant est prélevé (et conservé congelé à -80°C, dès qu'une lignée est établie) et remplacé par du milieu frais dans lequel sont émulsionnés les lymphocytes sédimentés. Quand une lignée est établie et la densité cellulaire suffisante dans un flacon, les cellules ainsi émulsionnées à la pipette sont réparties dans deux flacons et la culture ainsi dédoublée.

Les cellules lymphoïdes, après séparation sur Ficoll, sont mises en présence de cyclosporine A pendant 10 à 20 jours, afin de permettre l'inhibition et l'élimination des lymphocytes T de la culture. En effet, ceux-ci ont la capacité de bloquer l'immortalisation de lymphocytes B présents dans la culture par le virus EBV.

Selon les cas, la culture est maintenue telle que après traitement à la cyclosporine A, afin d'attendre la survenue éventuelle d'une immortalisation de lymphocyte B par la souche EBV hébergée par le patient lui-même (s'il est séropositif pour ce virus), ou bien 1 ml de surnageant de la lignée B95 productrice de virions EBV, après induction par les esters de phorbol et l'acide butyrique, est ajouté dans le flacon de culture afin de réaliser une infection massive des lymphocytes B présents par la souche EBV B95 et donc une immortalisation bien plus probable de quelques clones B.

Les flacons contenant les lymphocytes en suspension sont maintenus en culture au moins trois mois pendant lesquels la survenue de clones de lymphocytes B immortalisés, proliférant sous forme de "grappes" de cellules flottant dans le milieu de culture, est surveillée au microscope optique.

Lorsque des lignées lymphoblastoïdes sont établies, à partir de patients atteints de PR, elles sont régulièrement dédoublées et maintenues en culture continue. Les surnageants collectés deux fois par semaine à l'occasion des changements de milieu sont congelés à - 80°C pour une analyse ultérieure : activité transcriptase inverse, détection PCR du génomes des agents pathogènes et/ou infectants exprimés par les cellules B dans la culture, etc.. Des utilisations de ces cultures pour la mise en évidence et la caractérisation d'agents pathogènes et/ou infectants associés à la PR, seront présentées dans les exemples suivants.

### EXEMPLE 9 : OBTENTION DE CLONES MSRV-2 et MSRV-1 PAR AMPLIFICATION DES REGIONS POL CONSERVEES DES GENES D'ADN POLYMERASE-ARN DEPENDANTE SUR UNE PREPARATION D'AGENT PATHOGENE ET/OU INFECTANT PURIFIE A PARTIR DE CULTURE DE CELLULES DU LIQUIDE SYNOVIAL OU DE LIGNEE LYMPHOBLASTOÏDE DE PR.

L'approche moléculaire a consisté, comme dans l'exemple 1, à utiliser la technique PCR de Shih **(8)** qui permet d'amplifier une région relativement conservée du gène *pol* des rétrovirus exogènes et endogènes, mais aussi des virus codant pour une enzyme à activité transcriptase inverse (RT) tels que notamment le virus de l'hépatite B et, implicitement, de tout gène d'ADN polymérase ARN dépendante ou d'enzyme présentant des homologies de séquences suffisantes dans les régions définies par les amorces d'amplification utilisées. Cette technique PCR a été utilisée sur les acides nucléiques extraits d'une préparation d'agent infectant purifiée sur gradient selon le protocole décrit par Perron H. **(14)** à partir des surnageants d'une culture de cellules du liquide articulaire (LA) pathologique d'un cas de polyarthrire rhumatoïde (PR), obtenue selon le protocole décrit dans l'exemple 7. Les fractions contenant le pic d'activité RT mesurée selon les conditions optimisées pour la souche LM7 (7) sont reprises dans un volume d'un tampon contenant du thiocyanate de guanidine et sont stockées à -80°C jusqu'à extraction des acides nucléiques selon la technique décrite par Chomzynski P. **(15)**.

Préalablement à la réaction PCR, l'ARN de l'échantillon a été transcrit en ADN complémentaire (ADNc) avec des amorces dites "random" (hexanucléotides en mélange) à l'aide du Kit "ADNc synthesis system plus" (Amersham), selon les instructions du fabricant et en se basant sur une valeur approximative à un log près de la quantité d'ARN présente dans l'échantillon. Alternativement, la synthèse du ADNc peut s'effectuer directement en présence des amorces utilisées pour la PCR selon un protocole de "RT-PCR" en une étape décrit dans le document EP-A-0569272.

L'ADN obtenu après amplification PCR de l'ADNc, a été inséré dans un plasmide à l'aide du kit TA Cloning® (British Biotechnology), répliqué, extrait et séquencé selon les protocoles décrits dans les exemples 1 et 2.

Les séquences obtenues ont ensuite été analysées à l'aide des logiciels Mac Vector® et Geneworks® sur banque de données informatiques Genebank®, pour les séquences nucléiques, et Swiss Prot®, pour les séquences en acides aminés déduites des trames de lecture mises en évidence dans les séquences nucléiques. L'analyse des séquences obtenues à partir de l'échantillon de particules sédimentant au pic d'activité transcriptase inverse provenant des surnageants décongelés de culture de cellules du LA de PR, a mis en évidence deux types de séquences: un premier type de séquence, retrouvé dans la minorité des clones correspond à des séquences significativement homologues à la région "pol" équivalente du rétrovirus MSRV-1 préalablement isolé et caractérisé chez des patients atteints de SEP (demandes de brevet français 92 04322, 92 13447, 92 13443, 92 01529, 94 01530, 94 01531, 94 01532), et un second type de séquence, retrouvé dans la majorité des clones, qui correspond à des séquences très fortement homologues à la séquence attribuée à un agent infectant et/ou pathogène dénommé MSRV-2 préalablement isolé et caractérisé chez des patients atteints de SEP (demandes de brevet français 92 04322, 92 13447, 92 13443, 92 01529, 94 01530, 94 01531, 94 01532).

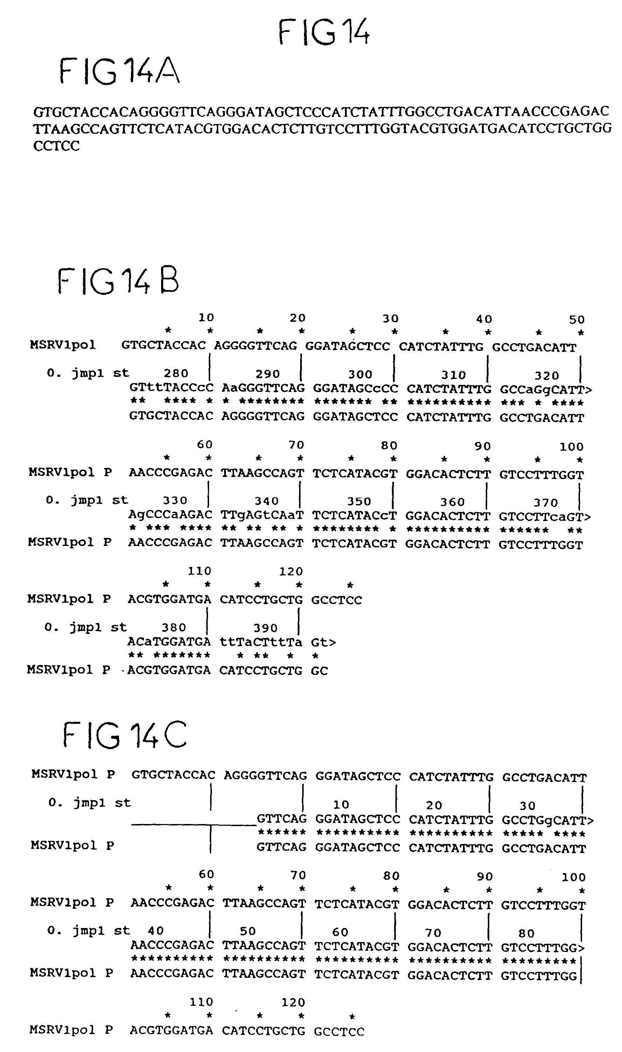

La comparaison entre une séquence de type MSRV-1 amplifiée et clonée à partir de matériel viral produit par une culture de cellules de LA de PR (clone "MSRV1polPR", SEQ ID N039) et les séquences de référence MSRV-1 clonées à partir de virion produit par des cellules provenant de patients atteints de SEP est présentée dans la figure 14.

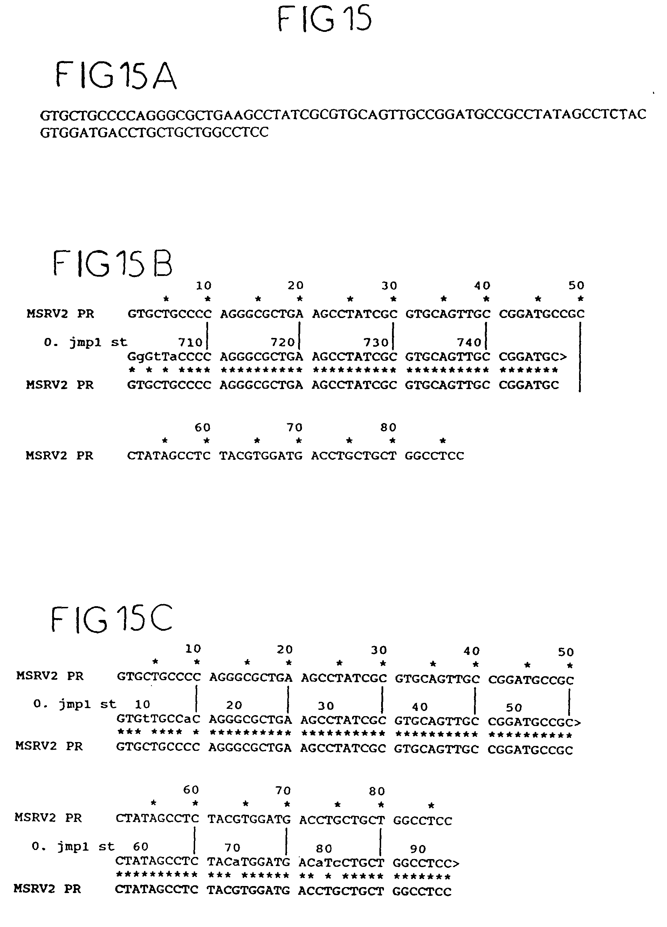

La comparaison entre une séquence de type MSRV-2 amplifiée et clonée à partir de matériel infectieux produit par une culture de cellules de LA de PR (clone "MSRV2sPR" SEQ ID N040)et les séquences de référence MSRV-2 clonées à partir de matériel infectieux produit par des cellules provenant de patients atteints de SEP est présentée dans la figure 15.

Les deux types de séquences retrouvées avec les amorces dégénérées "transcriptases inverses" de Shih et coll. **(8)** correspondent donc exactement à ce qui a été trouvé dans des conditions similaires, dans les cultures de cellules leptoméningées, de plexus choroïdes ou de lignées lymphoblastoïdes B provenant de différents patients atteints de SEP (demandes de brevet français 92 04322, 92 13447, 92 13443, 92 01529, 94 01530, 94 01531, 94 01532).

### EXEMPLE 10 : VERIFICATION, A L'AIDE D'AMORCES PCR SPECIFIQUES ET D'UNE HYBRIDATION SPECIFIQUE SELON LA TECHNIQUE ELOSA, DE LA PRESENCE DES SEQUENCES MSRV-1 ET MSRV2 DANS LES SURNAGEANTS DE CULTURE DE CELLULES ET, DIRECTEMENT, DANS LES FLUIDES BIOLOGIQUES DE PATIENTS ATTEINTS DE PR

Plusieurs techniques PCR ont été utilisées pour détecter les génomes MSRV-1 et MSRV-2 dans des surnageants de culture de cellules, des plasmas et des liquides articulaires de patients atteints de PR et de patients atteints d'autres maladies rhumatismales (non-PR).

L'extraction des ARN de plasma et de surnageant de cultures a été effectuée selon la technique décrite par P.Chomzynski **(15)**, après addition d'un volume du tampon contenant du guanidinium thiocyanate à 1 ml de plasma ou de surnageant de culture gardé congelé à -80°C après recueil.

L'extraction des ARN de liquide articulaire (LA) et de cellules mononuclées sanguines (lymphocytes et monocytes) a été effectuée selon le protocole suivant:
toutes les solutions sont réalisées dans de l'eau distillée traitée par le DEPC pour éliminer toute trace d'ARNase;
500 µl à 1 ml de LA sont décongelés et 1/100ème de volume d'une solution à 20% de SDS y est ajouté ainsi qu'une solution de protéinase K à raison de 7 µl par ml de LA; le liquide est brièvement vortexé et incubé à 37°C après addition de RNAsin (Boehringer) ou de RNA guard (Pharmacia); après 1 h d'incubation, 1 volume de tampon GUT selon Chomzynski **(15)** est ajouté et le mélange immédiatement vortexé. Par la suite, l'ARN présent dans le mélange est extrait selon le protocole précité décrit par Chomzynski **(15)**.

Pour la détection de MSRV2, la séquence MSRV-2EL1 (SEQ ID N012) a permis de définir plusieurs couples d'amorces oligonucléotidiques utilisables pour l'amplification d'ADN ou d'ARN spécifiques par la technique PCR.

Les premiers couples d'amorces MSRV2 définis ci-après ont permis de réaliser une détection spécifique du génome MSRV-2 dans différentes cellules humaines, par une étape de PCR, ou de RT-PCR selon un un procédé d'amplification d'ARN tel que décrit dans le document EP-A-0 569 272.

Les amorces utilisées sont les suivantes:

amorce 5', identifiée par SEQ ID N014
5'GTAGTTCGATGTAGAAAGCG 3'

amorce 3', identifiée par SEQ ID N015
5'GCATCCGGCAACTGCACG 3'

La PCR est effectuée selon une succession de 35 cycles enchaînant, après l'étape de synthèse de l'ADNc, 1 min, à 94°C, 1 min, à 54°C et 1 min, à 72°C.

Pour cet exemple, l'ARN total extrait de différents types de cellules **(15)**, sans traitement par la DNase, a été utilisé dans cette réaction RT-PCR, ce qui permet une détection d'ARN et d'ADN spécifiques du pathogène dans l'extrait d'acides nucléiques enrichi en ARN. En effet, notre expérience montre que de l'ADN spécifique de MSRV2 peut être facilement détecté dans les acides nucléiques extraits selon le protocole sus-cité normalement destiné à l'extraction d'ARN.

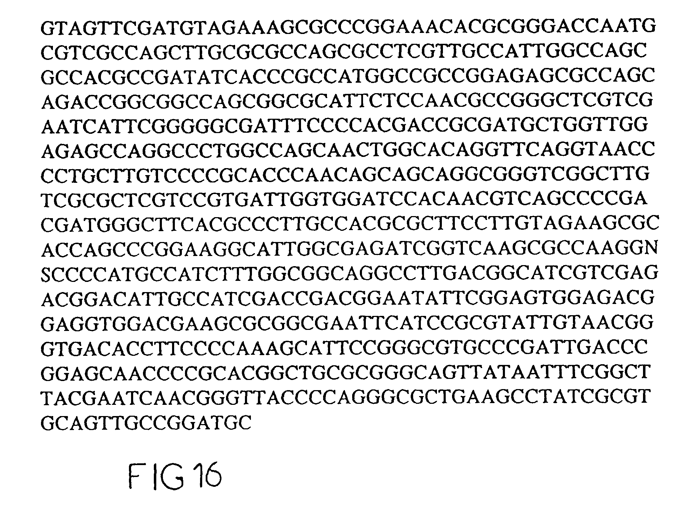

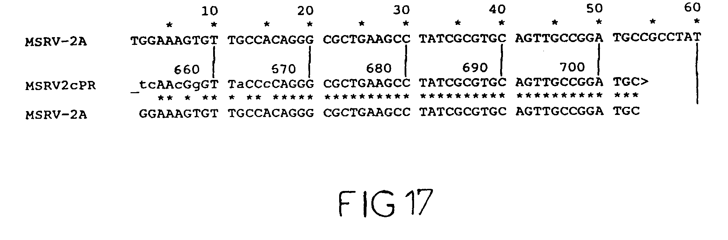

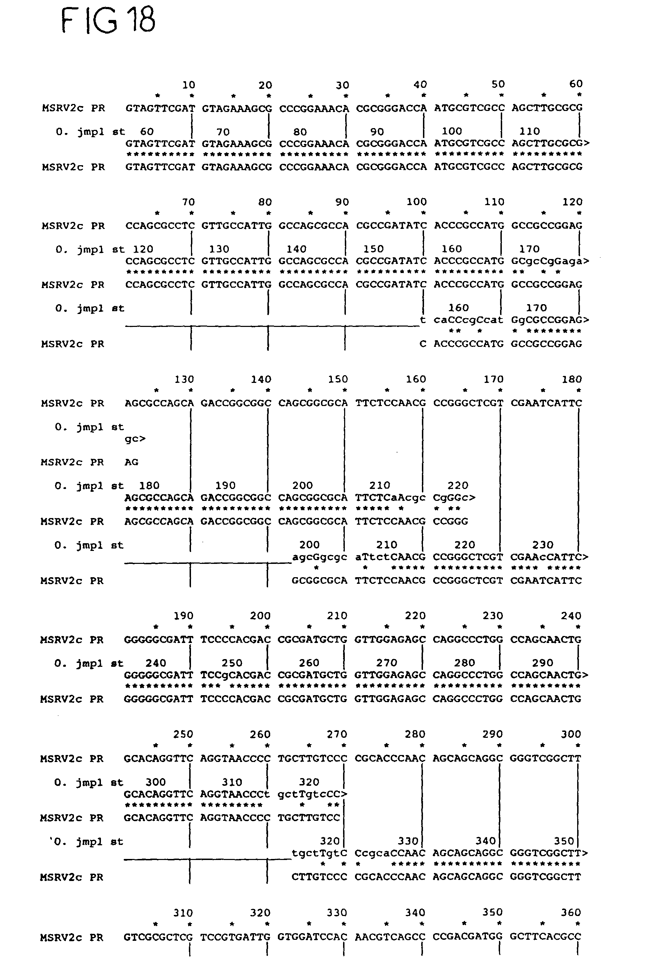

La séquence d'un clone "MSRV2cPR" amplifié selon cette technique avec les amorces PCR SEQ ID N014 et SEQ ID N015 à partir de cellules cultivées d'un liquide articulaire d'un patient atteint de PR est présentée dans la figure 16. Dans les figures 17 et 18, sont représentés les alignements de la séquence du clone "MSRV2cPR" avec les séquences nucléotidiques MSRV2 SEQ ID N010 et SEQ ID N012, respectivement, des clones obtenus chez des patients atteints de SEP. On peut constater que les séquences provenant d'échantillons de PR ou de SEP, sont significativement homologues.

Les nouveaux couples d'amorces MSRV2 définis ci-après ont permis de réaliser une détection spécifique du génome MSRV-2 dans différents fluides biologiques de patients, par deux étapes successives de PCR (dite "nichée"), ou de RT-PCR selon un un procédé d'amplification d'ARN tel que décrit dans le document EP-A-0 569 272, les séquences MSRV-2 pouvant être détectées sous forme d'ARN ou d'ADN dans les échantillons biologiques.

Cette PCR "nichée" est effectuée sur des échantillons d'acides nucléiques extraits selon ce qui a été décrit et n'ont pas été traités par la DNase.

Les amorces utilisées pour la première étape de 40 cycles avec une température d'hybridation de 48°C sont les suivantes:

amorce 5', identifiée par SEQ ID N044
5'GCCGATATCACCCGCCATGG 3'

amorce 3', identifiée par SEQ ID N015
5'GCATCCGGCAACTGCACG 3'

Après cette étape, 10 µl du produit d'amplification sont prélevés et utilisés pour réaliser une deuxième amplification PCR dite "nichée" avec des amorces situées à l'intérieur de la région déjà amplifiée. Cette deuxième étape se déroule sur 35 à 40 cycles, avec une température d'hybridation des amorces ("annealing") de 50°C. Le volume réactionnel est de 100 µl.

Les amorces utilisées pour la deuxième étape sont les suivantes:

amorce 5', identifiée par SEQ ID N045
5' CGCGATGCTGGTTGGAGAGC 3'

amorce 3', identifiée par SEQ ID N046
5' TCTCCACTCCGAATATTCCG 3'

Les résultats obtenus dans une série de PCR MSRV-2 effectuée selon la technique "PCR nested" sus-décrite sont présentés dans le tableau II annexé à la présente description.

Pour MSRV-1, l'amplification a été effectuée en deux étapes (PCR "nichée"), précédée d'une étape de synthèse de l'ADNc. De plus, l'échantillon d'acides nucléiques est préalablement traité par la DNase et un contrôle PCR sans RT (transcriptase inverse AMV) est effectué sur les deux étapes d'amplification de manière à vérifier que l'amplification RT-PCR provient exclusivement de l'ARN MSRV-1. En cas de contrôle sans RT positif, l'échantillon aliquoté d'ARN de départ est à nouveau traité par la DNase et amplifié à nouveau.

Le protocole de traitement par la DNase dépourvue d'activité RNase est le suivant: l'ARN extrait est aliquoté en présence de "RNAse inhibitor" (Boehringer-Mannheim) dans de l'eau traitée au DEPC à une concentration finale de 1 µg dans 10 µl; à ces 10 µl, est ajouté 1 µl de "RNAse-free DNASe" (Boehringer-Mannheim) et 1,2 µl de tampon à pH 5 contenant 0,1 M/l d'acétate de sodium et 5 mM/l de MgSO4; le mélange est incubé 15 min. à 20°C et porté à 95°C pendant 1,5 min. dans un "thermocycler".

La première étape de RT-PCR MSRV-1 peut être effectuée selon une variante du procédé d'amplification d'ARN tel que décrit dans le document EP-A-0 569 272. Notamment, l'étape de synthèse d'ADNc est effectuée à 42°C pendant une heure, mais la synthèse de l'ADNc est mieux réalisée en suivant le protocole suivant: l'ARN traité à la DNase et maintenu dans la glace est chauffé à 65°C pendant 10 min., puis aussitôt plongé dans la glace; il est ensuite ajouté à un mélange maintenu à 42°C dans le thermocycler destiné à la PCR et contenant, dans du tampon PCR 1X, du MgCl2 1,5 mM, chaque dNTP à 250 µM, chaque amorce (5' et 3' de la première étape de PCR) à 300-400 nM, 10 Unités de RT-AMV et 2,5 unités de Taq, dans un volume final de 100 µl; ce mélange est maintenu à 42°C pendant 1 à 2 h, puis porté à 95°C pendant 5 min. afin de dénaturer la RT-AMV. Les cycles d'amplification PCR débutent à la suite et se déroulent sur 40 cycles, avec une phase d'hybridation des amorces ("annealing") à 53°C pendant 1 min., une phase d'élongation à 72°C pendant 2 à 3 min. et une phase de dénaturation de l'ADN à 95°C pendant 1 min.

Les amorces utilisées pour cette première étape sont les suivantes:

amorce 5'
5'AGGAGTAAGGAAACCCAACGGAC 3' SEQID N°16

amorce 3'
5'TAAGAGTTGCACAAGTGCG 3' SEQID N°17

Après cette étape, 10 µl du produit d'amplification sont prélevés et utilisés pour réaliser une deuxième amplification PCR dite "nichée" avec des amorces situées à l'intérieur de la région déjà amplifiée. Les cycles d'amplification PCR se déroulent sur 35 à 40 cycles, avec une phase d'hybridation des amorces ("annealing") à 53°C pendant 1 min., une phase d'élongation à 72°C pendant 1 à 2 min., et une phase de dénaturation de l'ADN à 95°C pendant 1 min. La composition du mélange réactionnel est le même que pour l'étape précédente, à l'exeption de la RT-AMV qui n'y est pas ajoutée cette fois.

Les amorces utilisées pour cette deuxième étape sont les suivantes:

amorce 5', identifiée par SEQ ID NO18
5'TCAGGGATAGCCCCCATCTAT 3'

amorce 3', identifiée par SEQ ID N019
5'AACCCTTTGCCACTACATCAATTT 3'

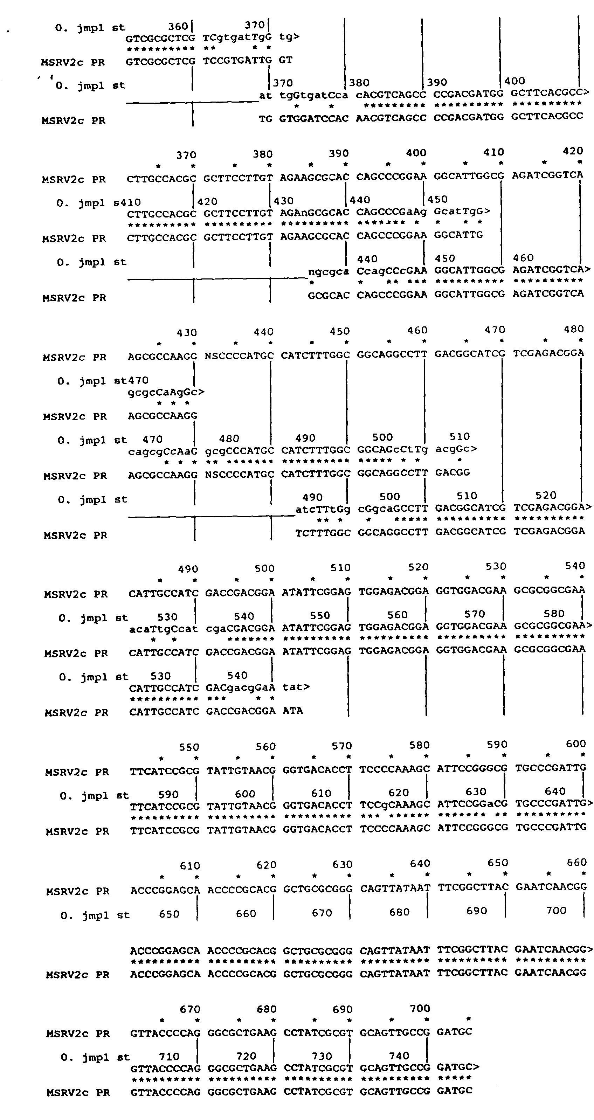

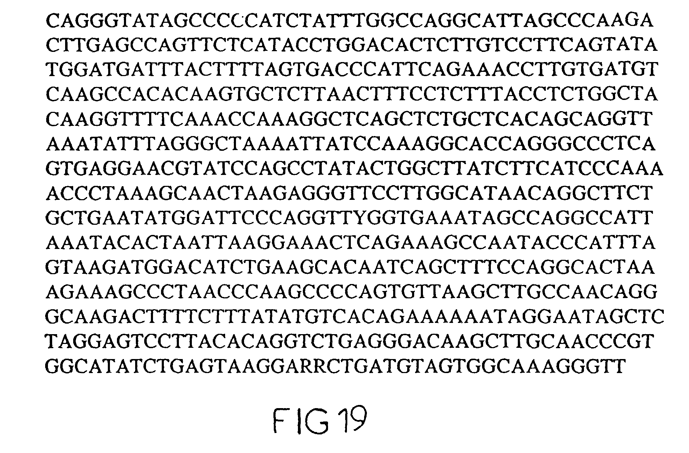

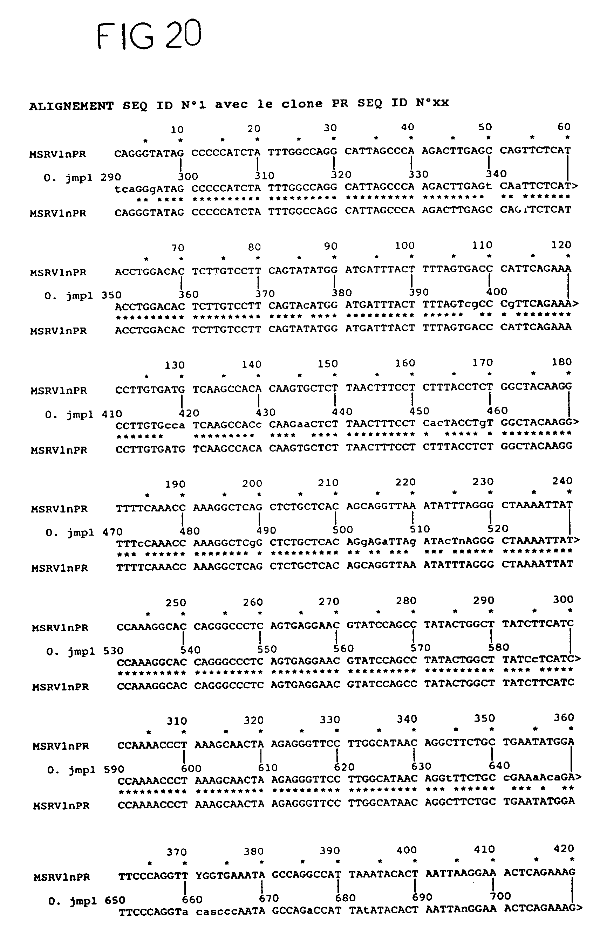

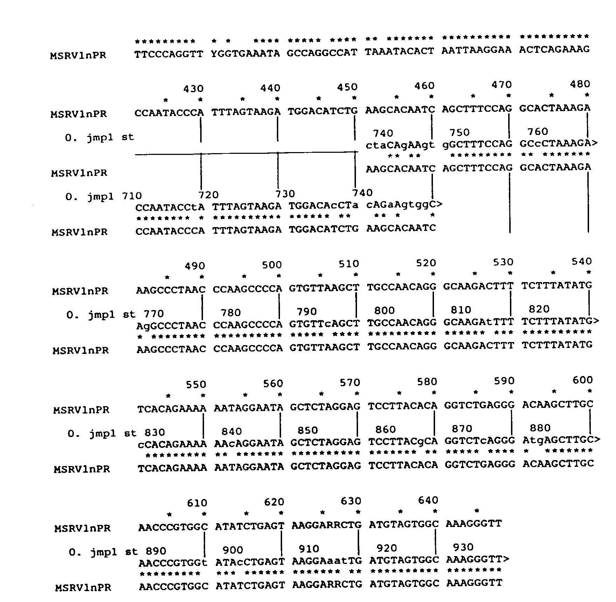

La séquence d'un clone "MSRV1nPR" amplifié selon la technique sus-décrite avec les amorces PCR SEQ ID N016 et SEQ ID N°17, puis SEQ ID N018 et SEQ ID N019 à partir d'ARN d'un liquide articulaire d'un patient atteint de PR est présentée dans la figure 19. Dans la figure 20, est représenté l'alignement de la séquence du clone "MSRV1nPR" avec la sequence nucléotidique MSRV1 SEQ ID N01 du clone de référence obtenu chez des patients atteints de SEP. On peut, ici aussi, constater que les séquences provenant de PR ou de SEP sont significativement homologues.

A ce jour, les autres séquences MSRV1 et MSRV2 amplifiées selon ces techniques PCR à partir d'échantillons de PR se sont aussi révélées identiques ou homologues aux séquences obtenues chez les patients atteints de SEP.

Dans une série de PCR MSRV-1 effectuée selon la technique "RT-PCR" nested sus-décrite, on a obtenu les résultats présentés dans le tableau I annexé à la présente dscription.

Le génome rétroviral MSRV-1 et le génome MSRV-2 sont donc bien retrouvés dans les prélèvements de fluides biologiques de patients atteints de PR. D'autres résultats obtenus sur des séries plus étendues confirment ces résultats.

Par ailleurs, la spécificité des séquences amplifiées par ces techniques PCR peut être vérifiée et évaluée par la technique "ELOSA" telle que décrite par F. Mallet **(22)** et dans le document FR-2 663 040.

Pour MSRV-1 les produits de la PCR nichée sus-décrite peuvent être testés dans deux systèmes ELOSA permettant de détecter séparément un consensus A et un consensus B + C + D de MSRV-1, correspondant aux sous-familles décrites dans l'exemple 2 et les figures 2, 3 et 4. En effet, les séquences proches du consensus B + C + D sont retrouvées essentiellement dans les échantillons d'ARN provenant de virions MSRV-1 purifiés à partir de cultures ou amplifiés dans des liquides biologiques extra-celluaires de patients PR ou SEP, alors que les séquences proches du consensus A sont essentiellement retrouvées dans l'ADN cellulaire humain normal.

Le système ELOSA/MSRV-1 pour la capture et l'hybridation spécifique des produits PCR de la sous-famille A utilise un oligonucléotide de capture cpV1A avec une liaison amine en 5' selon une technique décrite dans le document FR-A-2 663 040, et un oligonucléotide de détection dpVlA couplé à la peroxydase selon une technique décrite dans le document FR-A-2 663 040 (ou éventuellement couplé à la biotine) ayant respectivement pour séquence:

cpV1A 5' GATCTAGGCCACTTCTCAGGTCCAGS 3' SEQ ID N047

dpV1A 5' CATCTITTTGGICAGGCAITAGC 3' SEQ ID N048

Le système ELOSA/MSRV-1 pour la capture et l'hybridation spécifique des produits PCR de la sous-famille B+C+D utilise le même oligonucléotide de détection dpVlA couplé à la peroxydase selon la technique précédemment décrite (ou éventuellement couplé à la biotine) et un oligonucléotide de capture cpV1B avec une liaison amine en 5' selon la technique précédemment décrite ayant pour séquence:

cpV1B 5' CTTGAGCCAGTTCTCATACCTGGA 3' SEQ ID N049

Ainsi qu'on peut le voir dans le tableau I, une telle technique a été appliquée aux produits PCR amplifiés à partir des fluides biologiques de patients atteints de diverses maladies rhumatologiques : tous les patients atteints de PR qui présentaient une bande d'ADN amplifié de la taille attendue détectable sur gel d'agarose marqué au BET, étaient positifs pour MSRV1 type B + C + D et négatif pour MSRV1-A; un seul témoin non-PR présentait une bande amplifiée visible sur gel marqué au BET, d'une taille apparemment correcte ; le produit PCR correspondant s'est avèré négatif en ELOSA MSRV1-A et MSRV1-BCD, ainsi que l'ensemble des produits PCR des autre témoins non-PR; après clonage et séquençage de ce produit amplifié, il s'est avèré que celui-ci correspondait à une séquence artéfactuelle sans rapport avec MSRV1.

La technique ELOSA MSRV1 couplée à la RT-PCR nichée définie précédemment permet donc de réaliser un test de détection très sensible et très spécifique d'un rétrovirus MSRV 1 dans les fluides biologiques de patients.

Il est donc aussi envisageable, grâce aux découvertes effectuées et aux méthodes mises au point par les inventeurs dans le cadre de la polyarthrite rhumatoïde, de réaliser un diagnostic de l'infection et/ou de la réactivation MSRV-1 et/ou MSRV-2 et d'évaluer une thérapie dans la PR sur la base de son efficacité à "négativer" la détection de ces agents dans les fluides biologiques des patients. De plus, la détection précoce chez des personnes ne présentant pas encore de signes rhumatologiques de PR, pourrait permettre d'instaurer un traitement d'autant plus efficace sur l'évolution clinique ultérieure qu'il précèderait le stade lésionnel qui correspond à l'apparition de l'atteinte articulaire. Or, à ce jour, un diagnostic de PR ne peut être établi avant l'installation d'une symptomatologie inflammatoire voire lésionnelle et, donc, aucun traitement n'est instauré avant l'émergence d'une clinique évocatrice d'une atteinte articulaire déjà notable.

Le diagnostic d'une infection et/ou réactivation de MSRV-1 et/ou MSRV-2 chez l'homme est donc déterminant et la présente invention fourni les moyens d'un tel diagnostic aussi bien dans le cadre d'une symptomatologie neurologique (SEP) ou rhumatologique (PR) associées, ou encore dans le cadre d'une infection/réactivation pré-symptomatique ou non-encore associée à une clinique bien caractérisée.

Il est ainsi possible, outre de réaliser un diagnostic de l'infection et/ou de la réactivation MSRV-1 et/ou MSRV-2, d'évaluer une thérapie dans la SEP, la PR ou tout autre symptomatologie clinique associée sur la base de son efficacité à "négativer" la détection de ces agents dans les fluides biologiques des patients.

**TABLEAU I**

| | | | | | |
|---|---|---|---|---|---|
| Résultats de la détection par PCR suivie d'une hybridation selon la technique dite ELOSA, des génomes MSRV-1 dans les fluides biologiques de malades atteints de PR et d'autres maladies rhumatologiques. | | | | | |

| **Diagnostic** | **Effectif testé** | **Echantillon** | **RT-PCR Nichée MSRV-1 (bande BET)** | **ELOSA MSRV-1 sous type A** | **ELOSA MSRV-1 sous-type B (B+C+D)** |
|---|---|---|---|---|---|
| Témoin non-PR Rhumatologique | 10 | Milieu culture cellules LA | 10 - | 10 - | 10 - |
| | | | 0 + | 0 + | 0 + |
| | | | | | |
| PR | 10 | Milieu culture cellules LA | 5 - | 10 - | 5 - |
| | | | 5 + | 0 + | 5 + |
| Témoin non-PR rhumatologique | 8 | Liquide articulaire | 8 - | 8 - | 8 - |
| | | | 0 + | 0 + | 0 + |
| | | | | | |
| PR | 5 | Liquide articulaire | 3 - | 5 - | 3 - |
| | | | 2 + | 0 + | 2 + |
| Témoin non-PR rhumatologique | 7 | Plasma | 6 - | 7 - | 7 - |
| | | | 1 + | 0 + | 0 + |
| | | | | | |
| PR | 6 | Plasma | 4 - | 6 - | 4 - |
| | | | 2 + | 0 + | 2 + |
| Témoin non-PR rhumatologique | 4 | Cellules mono nuclées du sang (culot sec) | 4 - | 4 - | 4 - |
| | | | 0 + | 0 + | 0 + |
| | | | | | |
| PR | 2 | cellules mono nuclées du sang (culot sec) | 0 - | 2 - | 0 - |
| | | | 2 + | 0 + | 2 + |

**TABLEAU II**

| | | | | |
|---|---|---|---|---|
| Résultats de la détection par PCR suivie d'une hybridation selon la technique dite ELOSA, des génomes MSRV-2 dans les fluides biologiques de malades atteints de PR et d'autres maladies rhumatologiques. | | | | |

| **Diagnostic** | **Effectif testé** | **Echantillon** | **RT-PCR Nichée MSRV-2 (bande BET)** | **REMARQUES** |
|---|---|---|---|---|
| Témoin non-PR rhumatologique | 4 | Liquide articulaire | 4 - | |
| | | | 0 + | |
| PR | 5 | Liquide articulaire | 1 - | |
| | | | 4 + | |
| Témoin non-PR rhumatologique | 5 | Plasma | 4 - | patient positif = polytransfusé |
| | | | 1 + | |
| | | | | |
| PR | 8 | Plasma | 6 - | |
| | | | 2 + | |

LISTE DE SEQUENCES

(1) INFORMATIONS GENERALES:

(i) DEPOSANT:
(A) NOM: BIOMERIEUX
(B) RUE: AUCUNE
(C) VILLE: MARCY L'ETOILE
(E) PAYS: FRANCE
(F) CODE POSTAL: 69280

(ii) TITRE DE L' INVENTION: SEP EXTENSION

(iii) NOMBRE DE SEQUENCES: 49

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:

(i) CARACTERISTIQUES DE LA SEQUENCE:
(A) LONGUEUR: 1158 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 1:
CCCTTTGCCA CTACATCAAT TTTAGGAGTA AGGAAACCCA ACGGACAGTG GAGGTTAGTG 60
CAAGAACTCA GGATTATCAA TGAGGCTGTT GTTCCTCTAT ACCCAGCTGT ACCTAACCCT 120
TATACAGTGC TTTCCCAAAT ACCAGAGGAA GCAGAGTGGT TTACAGTCCT GGACCTTAAG 180
GATGCCTTTT TCTGCATCCC TGTACGTCCT GACTCTCAAT TCTTGTTTGC CTTTGAAGAT 240
CCTTTGAACC CAACGTCTCA ACTCACCTGG ACTGTTTTAC CCCAAGGGTT CAGGGATAGC 300
CCCCATCTAT TTGGCCAGGC ATTAGCCCAA GACTTGAGTC AATTCTCATA CCTGGACACT 360
CTTGTCCTTC AGTACATGGA TGATTTACTT TTAGTCGCCC GTTCAGAAAC CTTGTGCCAT 420
CAAGCCACCC AAGAACTCTT AACTTTCCTC ACTACCTGTG GCTACAAGGT TTCCAAACCA 480
AAGGCTCGGC TCTGCTCACA GGAGATTAGA TACTNAGGGC TAAAATTATC CAAAGGCACC 540
AGGGCCCTCA GTGAGGAACG TATCCAGCCT ATACTGGCTT ATCCTCATCC CAAAACCCTA 600
AAGCAACTAA GAGGGTTCCT TGGCATAACA GGTTTCTGCC GAAAACAGAT TCCCAGGTAC 660
ASCCCAATAG CCAGACCATT ATATACACTA ATTANGGAAA CTCAGAAAGC CAATACCTAT 720
TTAGTAAGAT GGACACCTAC AGAAGTGGCT TTCCAGGCCC TAAAGAAGGC CCTAACCCAA 780
GCCCCAGTGT TCAGCTTGCC AACAGGGCAA GATTTTTCTT TATATGCCAC AGAAAAAACA 840
GGAATAGCTC TAGGAGTCCT TACGCAGGTC TCAGGGATGA GCTTGCAACC CGTGGTATAC 900
CTGAGTAAGG AAATTGATGT AGTGGCAAAG GGTTGGCCTC ATNGTTTATG GGTAATGGNG 960
GCAGTAGCAG TCTNAGTATC TGAAGCAGTT AAAATAATAC AGGGAAGAGA TCTTNCTGTG1020
TGGACATCTC ATGATGTGAA CGGCATACTC ACTGCTAAAG GAGACTTGTG GTTGTCAGAC1080
AACCATTTAC TTAANTATCA GGCTCTATTA CTTGAAGAGC CAGTGCTGNG ACTGCGCACT1140
TGTGCAACTC TTAAACCC 1158

(2) INFORMATIONS POUR LA SEQ ID NO: 2:

(i) CARACTERISTIQUES DE LA SEQUENCE:
(A) LONGUEUR: 297 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 2:
CCCTTTGCCA CTACATCAAT TTTAGGAGTA AGGAAACCCA ACGGACAGTG GAGGTTAGTG 60
CAAGAACTCA GGATTATCAA TGAGGCTGTT GTTCCTCTAT ACCCAGCTGT ACCTAACCCT 120
TATACAGTGC TTTCCCAAAT ACCAGAGGAA GCAGAGTGGT TTACAGTCCT GGACCTTAAG 180
GATGCCTTTT TCTGCATCCC TGTACGTCCT GACTCTCAAT TCTTGTTTGC CTTTGAAGAT 240
CCTTTGAACC CAACGTCTCA ACTCACCTGG ACTGTTTTAC CCCAAGGGTT CAAGGGA 297

(2) INFORMATIONS POUR LA SEQ ID NO: 3:

(i) CARACTERISTIQUES DE LA SEQUENCE:
(A) LONGUEUR: 85 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 3:
GTTTAGGGAT ANCCCTCATC TCTTTGGTCA GGTACTGGCC CAAGATCTAG GCCACTTCTC 60
AGGTCCAGSN ACTCTGTYCC TTCAG 85

(2) INFORMATIONS POUR LA SEQ ID NO: 4:

(i) CARACTERISTIQUES DE LA SEQUENCE:
(A) LONGUEUR: 86 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 4:
GTTCAGGGAT AGCCCCCATC TATTTGGCCA GGCACTAGCT CAATACTTGA GCCAGTTCTC 60
ATACCTGGAC AYTCTYGTCC TTCGGT 86

(2) INFORMATIONS POUR LA SEQ ID NO: 5:

(i) CARACTERISTIQUES DE LA SEQUENCE:
(A) LONGUEUR: 85 paires de bases

(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 5:
GTTCARRGA TAGCCCCCATC TATTTGGCCW RGYATTAGCC CAAGACTTGA GYCAATTCTC 60
ATACCTGGA CACTCTTGTCC TTYRG 85

(2) INFORMATIONS POUR LA SEQ ID NO: 6:

(i) CARACTERISTIQUES DE LA SEQUENCE:
(A) LONGUEUR: 85 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 6:
GTTCAGGGAT AGCTCCCATC TATTTGGCCT GGCATTAACC CGAGACTTAA GCCAGTTCTY 60
ATACGTGGAC ACTCTTGTCC TTTGG 85

(2) INFORMATIONS POUR LA SEQ ID NO: 7:

(i) CARACTERISTIQUES DE LA SEQUENCE:
(A) LONGUEUR: 111 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 7:
GTGTTGCCAC AGGGGTTTAR RGATANCYCY CATCTMTTTG GYCWRGYAYT RRCYCRAKAY 60
YTRRGYCAVT TCTYAKRYSY RGSNAYTCTB KYCCTTYRGT ACATGGATGA C 111

(2) INFORMATIONS POUR LA SEQ ID NO: 8:

(i) CARACTERISTIQUES DE LA SEQUENCE:
(A) LONGUEUR: 645 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 8:
TCAGGGATAG CCCCCATCTA TTTGGCCAGG CATTAGCCCA AGACTTGAGT CAATTCTCAT 60
ACCTGGACAC TCTTGTCCTT CAGTACATGG ATGATTTACT TTTAGTCGCC CGTTCAGAAA 120

CCTTGTGCCA TCAAGCCACC CAAGAACTCT TAACTTTCCT CACTACCTGT GGCTACAAGG 180
TTTCCAAACC AAAGGCTCGG CTCTGCTCAC AGGAGATTAG ATACTNAGGG CTAAAATTAT 240
CCAAAGGCAC CAGGGCCCTC AGTGAGGAAC GTATCCAGCC TATACTGGCT TATCCTCATC 300
CCAAAACCCT AAAGCAACTA AGAGGGTTCC TTGGCATAAC AGGTTTCTGC CGAAAACAGA 360
TTCCCAGGTA CASCCCAATA GCCAGACCAT TATATACACT AATTANGGAA ACTCAGAAAG 420
CCAATACCTA TTTAGTAAGA TGGACACCTA CAGAAGTGGC TTTCCAGGCC CTAAAGAAGG 480
CCCTAACCCA AGCCCCAGTG TTCAGCTTGC CAACAGGGCA AGATTTTTCT TTATATGCCA 540
CAGAAAAAAC AGGAATAGCT CTAGGAGTCC TTACGCAGGT CTCAGGGATG AGCTTGCAAC 600
CCGTGGTATA CCTGAGTAAG GAAATTGATG TAGTGGCAAA GGGTT 645

(2) INFORMATIONS POUR LA SEQ ID NO: 9:

(i) CARACTERISTIQUES DE LA SEQUENCE:
(A) LONGUEUR: 741 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 9:
CAAGCCACCC AAGAACTCTT AAATTTCCTC ACTACCTGTG GCTACAAGGT TTCCAAACCA 60
AAGGCTCAGC TCTGCTCACA GGAGATTAGA TACTTAGGGT TAAAATTATC CAAAGGCACC 120
AGGGGCCTCA GTGAGGAACG TATCCAGCCT ATACTGGGTT ATCCTCATCC CAAAACCCTA 180
AAGCAACTAA GAGGGTTCCT TAGCATGATC AGGTTTCTGC CGAAAACAAG ATTCCCAGGT 240
ACAACCAAAA TAGCCAGACC ATTATATACA CTAATTAAGG AAACTCAGAA AGCCAATACC 300
TATTTAGTAA GATGGACACC TAAACAGAAG GCTTTCCAGG CCCTAAAGAA GGCCCTAACC 360
CAAGCCCCAG TGTTCAGCTT GCCAACAGGG CAAGATTTTT CTTTATATGG CACAGAAAAA 420
ACAGGAATCG CTCTAGGAGT CCTTACACAG GTCCGAGGGA TGAGCTTGCA ACCCGTGGCA 480
TACCTGAATA AGGAAATTGA TGTAGTGGCA AAGGGTTGGC CTCATNGTTT ATGGGTAATG 540
GNGGCAGTAG CAGTCTNAGT ATCTGAAGCA GTTAAAATAA TACAGGGAAG AGATCTTNCT 600
GTGTGGACAT CTCATGATGT GAACGGCATA CTCACTGCTA AAGGAGACTT GTGGTTGTCA 660
GACAACCATT TACTTAANTA TCAGGCTCTA TTACTTGAAG AGCCAGTGCT GNGACTGCGC 720
ACTTGTGCAA CTCTTAAACC C 741

(2) INFORMATIONS POUR LA SEQ ID NO: 10:

(i) CARACTERISTIQUES DE LA SEQUENCE:
(A) LONGUEUR: 93 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 10:
TGGAAAGTGT TGCCACAGGG CGCTGAAGCC TATCGCGTGC AGTTGCCGGA TGCCGCCTAT 60
AGCCTCTACA TGGATGACAT CCTGCTGGCC TCC 93

(2) INFORMATIONS POUR LA SEQ ID NO: 11:

(i) CARACTERISTIQUES DE LA SEQUENCE:
(A) LONGUEUR: 96 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 11:
TTGGATCCAG TGYTGCCACA GGGCGCTGAA GCCTATCGCG TGCAGTTGCC GGATGCCGCC 60
TATAGCCTCT ACGTGGATGA CCTSCTGAAG CTTGAG 96

(2) INFORMATIONS POUR LA SEQ ID NO: 12:

(i) CARACTERISTIQUES DE LA SEQUENCE:
(A) LONGUEUR: 748 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 12:
TGCAAGCTTC ACCGCTTGCT GGATGTAGGC CTCAGTACCG GNGTGCCCCG CGCGCTGTAG 60
TTCGATGTAG AAAGCGCCCG GAAACACGCG GGACCAATGC GTCGCCAGCT TGCGCGCCAG 120
CGCCTCGTTG CCATTGGCCA GCGCCACGCC GATATCACCC GCCATGGCGC CGGAGAGCGC 180
CAGCAGACCG GCGGCCAGCG GCGCATTCTC AACGCCGGGC TCGTCGAACC ATTCGGGGGC 240
GATTTCCGCA CGACCGCGAT GCTGGTTGGA GAGCCAGGCC CTGGCCAGCA ACTGGCACAG 300
GTTCAGGTAA CCCTGCTTGT CCCGCACCAA CAGCAGCAGG CGGGTCGGCT TGTCGCGCTC 360
GTCGTGATTG GTGATCCACA CGTCAGCCCC GACGATGGGC TTCACGCCCT TGCCACGCGC 420
TTCCTTGTAG ANGCGCACCA GCCCGAAGGC ATTGGCGAGA TCGGTCAGCG CCAAGGCGCC 480
CATGCCATCT TTGGCGGCAG CCTTGACGGC ATCGTCGAGA CGGACATTGC CATCGACGAC 540
GGAATATTCG GAGTGGAGAC GGAGGTGGAC GAAGCGCGGC GAATTCATCC GCGTATTGTA 600
ACGGGTGACA CCTTCCGCAA AGCATTCCGG ACGTGCCCGA TTGACCCGGA GCAACCCCGC 660
ACGGCTGCGC GGGCAGTTAT AATTTCGGCT TACGAATCAA CGGGTTACCC CAGGGCGCTG 720
AAGCCTATCG CGTGCAGTTG CCGGATGC 748

(2) INFORMATIONS POUR LA SEQ ID NO: 13:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 13:

GCATCCGGCA ACTGCACG 18

(2) INFORMATIONS POUR LA SEQ ID NO: 14:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 14:

GTAGTTCGAT GTAGAAAGCG 20

(2) INFORMATIONS POUR LA SEQ ID NO: 15:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 15:

GCATCCGGCA ACTGCACG 18

(2) INFORMATIONS POUR LA SEQ ID NO: 16:

(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 16:
AGGAGTAAGG AAACCCAACG GAC 23

(2) INFORMATIONS POUR LA SEQ ID NO: 17:

(i) CARACTERISTIQUES DE LA SEQUENCE:
(A) LONGUEUR: 19 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 17:
TAAGAGTTGC ACAAGTGCG 19

(2) INFORMATIONS POUR LA SEQ ID NO: 18:

(i) CARACTERISTIQUES DE LA SEQUENCE:
(A) LONGUEUR: 21 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 18:
TCAGGGATAG CCCCCATCTA T 21

(2) INFORMATIONS POUR LA SEQ ID NO: 19:

(i) CARACTERISTIQUES DE LA SEQUENCE:
(A) LONGUEUR: paires de bases
(B) TYPE: 24 nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 19:
AACCCTTTGC CACTACATCA ATTT 24

(2) INFORMATIONS POUR LA SEQ ID NO: 20:

(i) CARACTERISTIQUES DE LA SEQUENCE:
(A) LONGUEUR: 15 paires de bases
(B) TYPE: nucléotide
(C) NOMBRE DE BRINS: simple
(D) CONFIGURATION: linéaire

(ii) TYPE DE MOLECULE: ADNc

(ix) CARACTERISTIQUES:
(B) EMPLACEMENT: 5, 7, 10, 13
(D) AUTRES INFORMATIONS: G représente l'inosine (i)

(xi) DESCRIPTION DE LA SEQUENCE: SEQ ID NO: 20:
GGTCGTGCCG CAGGG 15

(2) INFORMATIONS POUR LA SEQ ID NO: 21:

(i) CARACTERISTIQUES DE LA SEQUENCE:
(A) LONGUEUR: 21 paires de bases

(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 21:
TTAGGGATAG CCCTCATCTC T 21

(2) INFORMATIONS POUR LA SEQ ID NO: 22:

(i) CARACTERISTIQUES DE LA SEQUENCE:
(A) LONGUEUR: 21 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 22:
TCAGGGATAG CCCCCATCTA T 21

(2) INFORMATIONS POUR LA SEQ ID NO: 23:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 23
AACCCTTTGC CACTACATCA ATTT 24

(2) INFORMATIONS POUR LA SEQ ID NO: 24:

(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 24
GCGTAAGGAC TCCTAGAGCT ATT 23

(2) INFORMATIONS POUR LA SEQ ID NO: 25:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 25
TCATCCATGT ACCGAAGG 18

(2) INFORMATIONS POUR LA SEQ ID NO: 26:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 26
ATGGGGTTCC CAAGTTCCCT 20

(2) INFORMATIONS POUR LA SEQ ID NO: 27:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 27
GCCGATATCA CCCGCCATGG 20

(2) INFORMATIONS POUR LA SEQ ID NO: 28:

(i) CARACTERISTIQUES DE LA SEQUENCE:
(A) LONGUEUR: 18 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 28
GCATCCGGCA ACTGCACG 18

(2) INFORMATIONS POUR LA SEQ ID NO: 29:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 29
CGCGATGCTG GTTGGAGAGC 20

(2) INFORMATIONS POUR LA SEQ ID NO: 30:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 30
TCTCCACTCC GAATATTCCG 20

(2) INFORMATIONS POUR LA SEQ ID NO: 31:

(i) CARACTERISTIQUES DE LA SEQUENCE:
(A) LONGUEUR: 26 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 31
GATCTAGGCC ACTTCTCAGG TCCAGS 26

(2) INFORMATIONS POUR LA SEQ ID NO: 32:

(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc

(ix) CARACTERISTIQUES:
(B) EMPLACEMENT: 6, 12, 19

(D) AUTRES INFORMATIONS: G représente l'inosine (i)

(xi) DESCRIPTION DE LA SEQUENCE: SEQ ID NO: 32
CATCTGTTTG GGCAGGCAGT AGC                                    23

2) INFORMATIONS POUR LA SEQ ID NO: 33:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 33
CTTGAGCCAG TTCTCATACC TGGA                                   24

2) INFORMATIONS POUR LA SEQ ID NO: 34:

(i) CARACTERISTIQUES DE LA SEQUENCE:
(A) LONGUEUR: 22 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 34
AGTGYTRCCM CARGGCGCTG AA                                     22

2) INFORMATIONS POUR LA SEQ ID NO: 35:

(i) CARACTERISTIQUES DE LA SEQUENCE:
(A) LONGUEUR: 22 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 35
GMGGCCAGCA GSAKGTCATC CA                                     22

2) INFORMATIONS POUR LA SEQ ID NO: 36:

(i) CARACTERISTIQUES DE LA SEQUENCE:
(A) LONGUEUR: 22 paires de bases
(B) TYPE: nucléotide
(C) NOMBRE DE BRINS: simple

(D) CONFIGURATION: linéaire

(ii) TYPE DE MOLECULE: ADNc

(xi) DESCRIPTION DE LA SEQUENCE: SEQ ID NO: 36
GGATGCCGCC TATAGCCTCT AC 22

2) INFORMATIONS POUR LA SEQ ID NO: 37:

(i) CARACTERISTIQUES DE LA SEQUENCE:
(A) LONGUEUR: 22 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 37
AAGCCTATCG CGTGCAGTTG CC 22

(2) INFORMATIONS POUR LA SEQ ID NO: 38:

(i) CARACTERISTIQUES DE LA SEQUENCE:
(A) LONGUEUR: 40 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 38:
TAAAGATCTA GAATTCGGCT ATAGGCGGCA TCCGGCAAGT 40

(2) INFORMATIONS POUR LA SEQ ID NO: 39:

(i) CARACTERISTIQUES DE LA SEQUENCE:
(A) LONGUEUR: 126 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 39:
GTGCTACCAC AGGGGTTCAG GGATAGCTCC CATCTATTTG GCCTGACATT AACCCGAGAC 60
TTAAGCCAGT TCTCATACGT GGACACTCTT GTCCTTTGGT ACGTGGATGA CATCCTGCTG 120
GCCTCC 126

(2) INFORMATIONS POUR LA SEQ ID NO: 40:

(i) CARACTERISTIQUES DE LA SEQUENCE:
(A) LONGUEUR: 87 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 40:
GTGCTGCCCC AGGGCGCTGA AGCCTATCGC GTGCAGTTGC CGGATGCCGC CTATAGCCTC 60
TACGTGGATG ACCTGCTGCT GGCCTCC 87

(2) INFORMATIONS POUR LA SEQ ID NO: 41:

(i) CARACTERISTIQUES DE LA SEQUENCE:
(A) LONGUEUR: 705 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 41:
GTAGTTCGAT GTAGAAAGCG CCCGGAAACA CGCGGGACCA ATGCGTCGCC AGCTTGCGCG 60
CCAGCGCCTC GTTGCCATTG GCCAGCGCCA CGCCGATATC ACCCGCCATG GCCGCCGGAG 120
AGCGCCAGCA GACCGGCGGC CAGCGGCGCA TTCTCCAACG CCGGGCTCGT CGAATCATTC 180
GGGGGCGATT TCCCCACGAC CGCGATGCTG GTTGGAGAGC CAGGCCCTGG CCAGCAACTG 240
GCACAGGTTC AGGTAACCCC TGCTTGTCCC CGCACCCAAC AGCAGCAGGC GGGTCGGCTT 300
GTCGCGCTCG TCCGTGATTG GTGGATCCAC AACGTCAGCC CCGACGATGG GCTTCACGCC 360
CTTGCCACGC GCTTCCTTGT AGAAGCGCAC CAGCCCGGAA GGCATTGGCG AGATCGGTCA 420
AGCGCCAAGG NSCCCCATGC CATCTTTGGC GGCAGGCCTT GACGGCATCG TCGAGACGGA 480
CATTGCCATC GACCGACGGA ATATTCGGAG TGGAGACGGA GGTGGACGAA GCGCGGCGAA 540
TTCATCCGCG TATTGTAACG GGTGACACCT TCCCCAAAGC ATTCCGGGCG TGCCCGATTG 600
ACCCGGAGCA ACCCCGCACG GCTGCGCGGG CAGTTATAAT TTCGGCTTAC GAATCAACGG 660
GTTACCCCAG GGCGCTGAAG CCTATCGCGT GCAGTTGCCG GATGC 705

(2) INFORMATIONS POUR LA SEQ ID NO: 42:

(i) CARACTERISTIQUES DE LA SEQUENCE:
(A) LONGUEUR: 648 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 42:
CAGGGTATAG CCCCCATCTA TTTGGCCAGG CATTAGCCCA AGACTTGAGC CAGTTCTCAT 60
ACCTGGACAC TCTTGTCCTT CAGTATATGG ATGATTTACT TTTAGTGACC CATTCAGAAA 120
CCTTGTGATG TCAAGCCACA CAAGTGCTCT TAACTTTCCT CTTTACCTCT GGCTACAAGG 180
TTTTCAAACC AAAGGCTCAG CTCTGCTCAC AGCAGGTTAA ATATTTAGGG CTAAAATTAT 240
CCAAAGGCAC CAGGGCCCTC AGTGAGGAAC GTATCCAGCC TATACTGGCT TATCTTCATC 300

CCAAAACCCT AAAGCAACTA AGAGGGTTCC TTGGCATAAC AGGCTTCTGC TGAATATGGA 360
TTCCCAGGTT YGGTGAAATA GCCAGGCCAT TAAATACACT AATTAAGGAA ACTCAGAAAG 420
CCAATACCCA TTTAGTAAGA TGGACATCTG AAGCACAATC AGCTTTCCAG GCACTAAAGA 480
AAGCCCTAAC CCAAGCCCCA GTGTTAAGCT TGCCAACAGG GCAAGACTTT TCTTTATATG 540
TCACAGAAAA AATAGGAATA GCTCTAGGAG TCCTTACACA GGTCTGAGGG ACAAGCTTGC 600
AACCCGTGGC ATATCTGAGT AAGGARRCTG ATGTAGTGGC AAAGGGTT 648

(2) INFORMATIONS POUR LA SEQ ID NO: 43:

(i) CARACTERISTIQUES DE LA SEQUENCE:
(A) LONGUEUR: 648 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 43:
CAGGGTATAG CCCCCATCTA TTTGGCCAGG CATTAGCCCA AGACTTGAGC CAGTTCTCAT 60
ACCTGGACAC TCTTGTCCTT CAGTATATGG ATGATTTACT TTTAGTGACC CATTCAGAAA 120
CCTTGTGATG TCAAGCCACA CAAGTGCTCT TAACTTTCCT CTTTACCTCT GGCTACAAGG 180
TTTTCAAACC AAAGGCTCAG CTCTGCTCAC AGCAGGTTAA ATATTTAGGG CTAAAATTAT 240
CCAAAGGCAC CAGGGCCCTC AGTGAGGAAC GTATCCAGCC TATACTGGCT TATCTTCATC 300
CCAAAACCCT AAAGCAACTA AGAGGGTTCC TTGGCATAAC AGGCTTCTGC TGAATATGGA 360
TTCCCAGGTT YGGTGAAATA GCCAGGCCAT TAAATACACT AATTAAGGAA ACTCAGAAAG 420
CCAATACCCA TTTAGTAAGA TGGACATCTG AAGCACAATC AGCTTTCCAG GCACTAAAGA 480
AAGCCCTAAC CCAAGCCCCA GTGTTAAGCT TGCCAACAGG GCAAGACTTT TCTTTATATG 540
TCACAGAAAA AATAGGAATA GCTCTAGGAG TCCTTACACA GGTCTGAGGG ACAAGCTTGC 600
AACCCGTGGC ATATCTGAGT AAGGARRCTG ATGTAGTGGC AAAGGGTT 648

(2) INFORMATIONS POUR LA SEQ ID NO: 44:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 44:
GCCGATATCA CCCGCCATGG 20

(2) INFORMATIONS POUR LA SEQ ID NO: 45:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 45:
CGCGATGCTG GTTGGAGAGC 20

(2) INFORMATIONS POUR LA SEQ ID NO: 46:

(i) CARACTERISTIQUES DE LA SEQUENCE:
(A) LONGUEUR: 20 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 46:
TCTCCACTCC GAATATTCCG 20

(2) INFORMATIONS POUR LA SEQ ID NO: 47:

(i) CARACTERISTIQUES DE LA SEQUENCE:
(A) LONGUEUR: 26 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 47:
GATCTAGGCC ACTTCTCAGG TCCAGS 26

(2) INFORMATIONS POUR LA SEQ ID NO: 48:

(i) CARACTERISTIQUES DE LA SEQUENCE:
(A) LONGUEUR: 23 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 48:
CATCTITTTG GICAGGCAIT AGC 23

(2) INFORMATIONS POUR LA SEQ ID NO: 49:

(i) CARACTERISTIQUES DE LA SEQUENCE:
(A) LONGUEUR: 24 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 49:
CTTGAGCCAG TTCTCATACC TGGA 24

### BIBLIOGRAPHIE

**(1) Sauvezio B.** et coll., dans "Polyarthrite rhumatoïde, aspects actuels et perspectives", Sany J., 1-13, Collection Médecine-Sciences Flammarion, Paris, 1987
**(2) Kahen A.**, Virus et polyarthrite rhumatoïde, dans "Polyarthrite rhumatoïde, aspects actuels et perspectives", Sany J., 1-13, collection Médecine-Sciences Flammarion, Paris, 1987
**(3) Fujinami R.S.** et Oldstone M.B.A., ed. Current Topics in Microbiology and Immunology, 145, Berlin, Springer Verlaq, 1989
**(4) Acha-Orbea H.** et Palmer E., Mls -a retrovirus exploits the immune system- Immunology Today 1991 ; 12, 271-276
**(5) Cole B.C.** et Atkin C.L., The mycoplasma arthritidis T-cell mitogen, MAM : a model superantigen, Immunology Today 1991 ; 12, 271-276
**(6) Posnet D.N.**, Do superantigens play a role in auto-immunity ? Semin. immunol. 1993 ; 5, 65-72
**(7) Perron H.** et coll., Res. Virol. 1989 ; 140, 551-561
**(8) Shih A.**, Misra R. et Rush M.G., Res. Virol. 1989 ; 63, 64-75
**(9) Fields** et Knipe, Fondamental Virology 1986, Rev Press N.Y.
**(10) Nielsen P.E.** et coll, Science 1991; 254, 1497-1500
**(11) Maniatis** et al, Molecular Cloning, Cold Spring Harbor, 1982
**(12) Southern. E.M.**, J. Mol. Biol. 1975 ; 98, 503
**(13) Dunn A.R.** et Hassel J.A., Cell 1977 ; 12, 23
**(14) Perron H.** et coll., Res. Vir. 1992 ; 143, 337-350
**(15) Chomzynski P.** et Sacchi N., Analytical Biochemistry 1987; 162, 156-159
**(16) Sambrook J.,** Fritsch E.F., et Maniatis T., Molecular cloning, a laboratory manual, Cold Spring Harbor, Laboratory Press, 1989
**(17) Meyerhans** et coll., Cell 1989 ; 58, 901-910
**(18) Linial M.L.** and Miller A.D., "Current topics in microbiology and immunobiology. Retroviruses, strategies of replication" vol. 157, 125-152; Swanstrom R. et Vogt P.K., éditeurs, Springer-Verlag, Heidelberg 1990
**(19) Lori F.** et coll., J. Virol. 1992 ; 66, 5067-5074
**(20) La Mantia** et coll., Nucleic Acids Research 1991 ; 19, 1513-1520
**(21) Frohman** et coll., Proc. Natl. Acad. Sci. USA 1988 ; 85, 8998-9002
**(22) Mallet F.** et coll., Journal of Clinical Microbiology 1993 ; 31, 1444-1449

## Revendications

1. Utilisation d'un matériel viral, à l'état purifié ou isolé, possédant une activité transcriptase inverse, apparenté à une famille d'éléments rétroviraux endogènes, tel qu'issu d'une souche virale possédant une activité transcriptase inverse, choisie parmi les souches dénommées respectivement POL-2, déposée le 22.07.1992 auprès de l'ECACC sous le numéro d'accès V92072202, et MS7PG déposée le 08.01.93 auprès de l'ECACC sous le numéro d'accès V93010816, et parmi leurs souches variantes consistant en des virus comprenant au moins un antigène qui est reconnu par au moins un anticorps dirigé contre au moins un antigène correspondant de l'un ou l'autre des virus des souches virales POL-2 et MS7PG précitées, pour obtenir une composition diagnostique, prophylactique ou thérapeutique pour détecter, prévenir ou traiter une infection par ledit matériel viral associé à la polyarthrite rhumatoïde, ou une réactivation dudit matériel.

2. Utilisation d'un matériel viral, à l'état purifié ou isolé, possédant une activité transcriptase inverse, apparenté à une famille d'éléments rétroviraux endogènes, tel que produit par une lignée cellulaire choisie parmi les lignées dénommées respectivement PLI-2 déposée le 22.07.1992 auprès de l'ECACC sous le numéro d'accès 92072201, et LM7PC déposée le 08.01.93 auprès de l'ECACC sous le numéro d'accès 93010817, ou par toute culture cellulaire infectée susceptible de produire un virus comprenant au moins un antigène qui est reconnu par au moins un anticorps dirigé contre au moins un antigène correspondant de l'un ou l'autre des virus produits par les lignées PLI-2 et LM7PC précitées, pour obtenir une composition diagnostique, prophylactique ou thérapeutique, pour détecter, prévenir ou traiter une infection par ledit matériel viral associé à la polyarthrite rhumatoïde, ou une réactivation dudit matériel.

3. Utilisation d'un matériel viral dont le génome comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 leurs séquences complémentaires, et leurs séquences équivalentes, notamment les séquences nucléotidiques présentant, pour toute suite de 100 monomères contigus, au moins 50 % et préférentiellement au moins 70 % d'homologie avec une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 et leurs séquences complémentaires, pour obtenir une composition diagnostique, prophylactique ou thérapeutique pour détecter, prévenir ou traiter une infection par ledit matériel viral associé à la polyarthrite rhumatoïde, ou une réactivation dudit matériel.

4. Utilisation d'un matériel rétroviral, dont le gène pol de son génome comprend une séquence nucléotidique équivalente, et notamment présentant au moins 50 % d'homologie, de préférence au moins 65% d'homologie avec une séquence nucléotidique appartenant au gène pol du génome du rétrovirus ERV-9 ou HSERV-9, pour obtenir une composition diagnostique, prophylactique ou thérapeutique, pour détecter, prévenir ou traiter une infection par ledit matériel viral associé à la polyarthrite rhumatoïde, ou une réactivation dudit matériel.

5. Utilisation d'un matériel rétroviral dont le gène *pol* de son génome code pour une séquence peptidique présentant au moins 50%, et de préférence au moins 70% d'homologie avec une séquence peptidique codée par le gène *pol* du génome du rétrovirus ERV-9 ou HSERV-9, pour obtenir une composition diagnostique, prophylactique ou thérapeutique, pour détecter, prévenir ou traiter une infection par ledit matériel viral associé à la polyarthrite rhumatoïde, ou une réactivation dudit matériel.

6. Utilisation d'un matériel rétroviral dont le gène *pol* de son génome code pour une séquence peptidique présentant, pour toute suite contigue d'au moins 30 acides aminés, au moins 50% et de préférence au moins 70% d'homologie avec une séquence peptidique codée par une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 et leurs séquences complémentaires, pour obtenir une composition diagnostique, prophylactique ou thérapeutique, pour détecter, prévenir ou traiter une infection par ledit matériel viral associé à la polyarthrite rhumatoïde, ou une réactivation dudit matériel.

7. Utilisation d'un fragment nucléotidique, dont la séquence nucléotidique comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 leurs séquences complémentaires, et leurs séquences équivalentes, notamment les séquences nucléotidiques présentant, pour toute suite de 100 monomères contigus, au moins 50%, et de préférence au moins 70% d'homologie, avec une séquence choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043 et leurs séquences complémentaires, pour obtenir une composition diagnostique, prophylactique ou thérapeutique, pour détecter, prévenir ou traiter une infection par ledit matériel viral associé à la polyarthrite rhumatoïde, ou une réactivation dudit matériel.

8. Utilisation d'une amorce spécifique comprenant une séquence nucléotidique identique ou équivalente à au moins une partie de la séquence nucléotidique d'un fragment défini à la revendication 7, notamment une séquence nucléotidique présentant, pour toute suite de 10 monomères contigus, au moins 70% d'homologie avec au moins une partie dudit fragment.pour l'amplification par polymérisation d'un ARN ou d'un ADN d'un matériel viral associé à la polyarthrite rhumatoïde.

9. Utilisation selon la revendication 8, caractérisée en ce que l'amorce comprend une séquence nucléotidique choisie parmi SEQ ID N016, SEQ ID N017, SEQ ID N018, SEQ ID N019, SEQ ID N020, SEQ ID N021, SEQ ID N022, SEQ ID N023, SEQ ID N024, SEQ ID N025, SEQ ID N026, SEQ ID N031, SEQ ID N032, SEQ ID N033, SEQ ID N047, SEQ ID N048, SEQ ID N049 et leurs séquences complémentaires.

10. Utilisation d'une sonde comprenant une séquence nucléotidique identique ou équivalente à au moins une partie de la séquence nucléotidique d'un fragment défini à la revendication 6, notamment une séquence nucléotidique présentant, pour toute suite de 10 monomères contigus, au moins 70% d'homologie avec au moins une partie dudit fragment pour obtenir une composition pour détecter, séparer, ou identifier, dans un échantillon biologique, un matériel viral associé à la polyarthrite rhumatoïde.

11. Utilisation selon la revendication 10, caractérisée en ce que la sonde comprend une séquence nucléotidique choisie parmi SEQ ID N03, SEQ ID N04, SEQ ID N05, SEQ ID N06, SEQ ID N07, SEQ ID N016, SEQ ID N017, SEQ ID N018, SEQ ID N019, SEQ ID N020, SEQ ID N021, SEQ ID N022, SEQ ID N023, SEQ ID N024, SEQ ID N025, SEQ ID N026, SEQ ID N031, SEQ ID N032, SEQ ID N033, SEQ ID N047, SEQ ID N048, SEQ ID N049 et leurs séquences complémentaires.

12. Utilisation d'un agent pathogène et/ou infectant, à l'état purifié ou isolé, différent du matériel viral défini à l'une quelconque des revendications 1 à 6, tel qu'issu d'une souche virale choisie parmi les souches dénommées respectivement POL-2, déposée le 22.07.1992 auprès de l'ECACC sous le numéro d'accès V92072202 et MS7PG déposée le 08.01.93 auprès de l'ECACC sous le numéro d'accès V93010816, et parmi leurs souches variantes, consistant en des agents pathogènes et/ou infectants comprenant au moins un antigène qui est reconnu par au moins un anticorps dirigé contre au moins un antigène correspondant de l'un ou l'autre des agents pathogènes et/ou infectants des souches virales POL-2 et MS7PG précitées, respectivement différents de l'un ou l'autre matériel rétroviral desdites souches, pour obtenir une composition diagnostique, prophylactique ou thérapeutique pour détecter, prévenir ou traiter une infection par ledit agent pathogène et/ou infectant associé à la polyarthrite rhumatoïde, ou une réactivation dudit agent.

13. Utilisation d'un agent pathogène et/ou infectant, à l'état purifié ou isolé, différent du matériel viral défini à l'une quelconque des revendications 1 à 6, tel que produit par une lignée cellulaire choisie parmi les lignées dénommées respectivement PLI-2 déposée le 22.07.1992 auprès de l'ECACC sous le numéro d'accès 92072201 et LM7PC déposée le 08.01.93 auprès de l'ECACC sous le numéro d'accès 93010817, et par toutes cultures cellulaires infectées susceptibles de produire au moins l'un ou l'autre des agents pathogènes et/ou infectants, et/ou leurs variants, ou par toute culture cellulaire infectée susceptible de produire un agent pathogène et/ou infectant comprenant au moins un antigène qui est reconnu par au moins un anticorps dirigé contre au moins un antigène correspondant de l'un ou l'autre des agents pathogènes et/ou infectants produits par les lignées PLI-2 et LM7PC précitées, pour obtenir une composition diagnostique, prophylactique ou thérapeutique pour détecter, prévenir ou traiter une infection par ledit agent pathogène et/ou infectant associé à la polyarthrite rhumatoïde, ou une réactivation dudit agent.

14. Utilisation d'un agent pathogène et/ou infectant comprenant un acide nucléique comprenant une séquence nucléotidique choisie parmi SEQ ID N010, SEQ ID N011, SEQ ID N012, SEQ ID N040, SEQ ID N041, leurs séquences complémentaires, et leurs séquences équivalentes, notamment les séquences nucléotidiques, présentant au moins 70% et préférentiellement au moins 90% d'homologie avec une séquence nucléotidique comprenant une séquence choisie parmi SEQ ID N010, SEQ ID N011, SEQ ID N012 SEQ ID N040, SEQ ID N041, et leurs séquences complémentaires, pour obtenir une composition diagnostique, prophylactique ou thérapeutique pour détecter, prévenir ou traiter une infection par ledit agent pathogène et/ou infectant associé à la polyarthrite rhumatoïde, ou une réactivation dudit agent.

15. Utilisation d'un fragment nucléotidique, comprenant une séquence nucléotidique choisie parmi SEQ ID N010, SEQ ID N011, SEQ ID N012, SEQ ID N040, SEQ ID N041, leurs séquences complémentaires, et leurs séquences équivalentes, notamment les séquences nucléotidiques présentant, pour toute suite de 100 monomères contigus, au moins 70%, et de préférence au moins 90% d'homologie, avec une séquence choisie parmi SEQ ID N010, SEQ ID N011, SEQ ID N012, SEQ ID N040, SEQ ID N041, et leurs séquences complémentaires, pour obtenir une composition diagnostique, prophylactique ou thérapeutique pour détecter, prévenir ou traiter une infection par ledit agent pathogène et/ou infectant associé à la polyarthrite rhumatoïde, ou une réactivation dudit agent.

16. Utilisation d'une amorce comprenant une séquence nucléotidique identique ou équivalente à au moins une partie de la séquence nucléotidique d'un fragment défini à la revendication 15, notamment une séquence nucléotidique présentant, pour toute suite de 10 monomères contigus, au moins 90% d'homologie avec au moins une partie dudit fragment, pour l'amplification par polymérisation d'un ARN ou d'un ADN d'un agent pathogène et/ou infectant associé à la polyarthrite rhumatoïde.

17. Utilisation selon la revendication 16, caractérisée en ce que l'amorce comprend une séquence nucléotidique choisie parmi SEQ ID N013, SEQ ID N014, SEQ ID N015, SEQ ID N027, SEQ ID N028, SEQ ID N029, SEQ ID NO30, SEQ ID N034, SEQ ID N035, SEQ ID N036, SEQ ID N037, SEQ ID N044, SEQ ID N045, SEQ ID N046 et leurs séquences complémentaires.

18. Utilisation d'une sonde comprenant une séquence nucléotidique identique ou équivalente à au moins une partie de la séquence nucléotidique d'un fragment selon la revendication 15, notamment une séquence nucléotidique présentant, pour toute suite de 10 monomères contigus, au moins 90% d'homologie avec au moins une partie dudit fragment, pour obtenir une composition diagnostique, prophylactique ou thérapeutique pour détecter, prévenir ou traiter une infection par ledit agent pathogène et/ou infectant associé à la polyarthrite rhumatoïde, ou une réactivation dudit agent.

19. Utilisation selon la revendication 18, caractérisée en ce que la sonde comprend une séquence nucléotidique choisie parmi SEQ ID N010, SEQ ID N011, SEQ ID N013, SEQ ID N014, SEQ ID N015, SEQ ID N027, SEQ ID N028, SEQ ID N029, SEQ ID N030, SEQ ID N034, SEQ ID N035, SEQ ID N036, SEQ ID N037, SEQ ID N044, SEQ ID N045, SEQ ID N046 et leurs séquences complémentaires.

20. Utilisation d'une association comprenant deux agents pathogènes et/ou infectants, à l'état isolé ou purifié, à savoir, un premier agent qui consiste en un virus humain, possédant une activité transcriptase inverse, et apparenté à une famille d'éléments rétroviraux endogènes, ou un variant dudit virus, et un second agent, ou un variant dudit second agent, ces deux agents pathogènes et/ou infectants étant tels que ceux issus d'une même souche virale choisie parmi les souches dénommées respectivement POL-2, déposée le 22.07.1992 auprès de l'ECACC sous le numéro d'accès V92072202 et MS7PG déposée le 08.01.93 auprès de l'ECACC sous le numéro d'accès V93010816, et parmi leurs souches variantes, pour obtenir une composition diagnostique, prophylactique ou thérapeutique pour détecter, prévenir ou traiter une infection par un premier agent pathogène et/ou infectant, et un second agent pathogène et/ou infectant, associés à la polyarthrite rhumatoïde, ou une réactivation de l'un ou l'autre dudit premier agent et dudit second agent.

21. Utilisation d'une association comprenant deux agents pathogènes et/ou infectants, à l'état isolé ou purifié, à savoir, un premier agent consistant en un virus humain possédant une activité transcriptase inverse, et apparenté à une famille d'éléments rétroviraux endogènes, ou un variant dudit virus, et un second agent, ou un variant dudit second agent, ces deux agents pathogènes et/ou infectants étant tels que ceux produits par une même lignée cellulaire choisie parmi les lignées dénommées respectivement PLI-2 déposée le 22.07.1992 auprès de l'ECACC sous le numéro d'accès 92072201 et LM7PC déposée le 08.01.93 auprès de l'ECACC sous le numéro d'accès 93010817, et par toutes cultures cellulaires infectées susceptibles de produire au moins l'un ou l'autre des agents pathogènes et/ou infectants, et/ou leurs variants, pour obtenir une composition diagnostique, prophylactique ou thérapeutique pour détecter, prévenir ou traiter une infection par un premier agent pathogène et infectant, et par un second agent pathogène et/ou infectant, associés à la polyarthrite rhumatoïde, ou une réactivation de l'un ou l'autre dudit premier agent et dudit second agent.

22. Utilisation d'une association comprenant deux agents pathogènes et/ou infectants, à l'état isolé ou purifié, à savoir un premier agent consistant en un virus, ou un variant dudit virus, dont le génome comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, leurs séquences complémentaires, et leurs séquences équivalentes, notamment les séquences nucléotidiques présentant, pour toute suite de 100 monomères contigus, au moins 50% et préférentiellement au moins 70% d'homologie avec une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, et leurs séquences complémentaires, et un second agent pathogène et/ou infectant, dont le génome comprend une séquence nucléotidique choisie parmi SEQ ID N010, SEQ ID N011, SEQ ID N012, SEQ ID N040, SEQ ID N041, leurs séquences complémentaires, et leurs séquences équivalentes, notamment les séquences nucléotidiques présentant, pour toute suite de 100 monomères contigus, au moins 70% et préférentiellement au moins 90% d'homologie avec une séquence nucléotidique choisie parmi SEQ ID N010, SEQ ID N011, SEQ ID N012, SEQ ID N040, SEQ ID N041, et leurs séquences complémentaires, pour obtenir une composition diagnostique, prophylactique ou thérapeutique pour détecter, prévenir ou traiter une infection par un premier agent pathogène et/ou infectant, et par un second agent pathogène et/ou infectant, associés à la polyarthrite rhumatoïde, ou une réactivation de l'un ou l'autre dudit premier agent et dudit second agent.

23. Utilisation d'une association de fragments nucléotidiques comprenant un premier fragment dont la séquence nucléotidique comprend une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, leurs séquences complémentaires, et leurs séquences équivalentes, notammment les séquences nucléotidiques présentant, pour toute suite de 100 monomères contigus, au moins 50% et de préférence au moins 70% d'homologie avec une séquence nucléotidique choisie parmi SEQ ID N01, SEQ ID N02, SEQ ID N03, SEQ ID N04, SEQ ID N05, SEQ ID N06, SEQ ID N07, SEQ ID N08, SEQ ID N09, SEQ ID N039, SEQ ID N042, SEQ ID N043, et leurs séquences complémentaires, et un second fragment dont la séquence nucléotidique comprend une séquence nucléotidique choisie parmi SEQ ID N010, SEQ ID N011, SEQ ID N012, SEQ ID N040, SEQ ID N041, leurs séquences complémentaires, et leurs séquences équivalentes, notammment les séquences nucléotidiques présentant, pour toute suite de 100 monomères contigus, au moins 70% et de préférence au moins 90% d'homologie avec une séquence nucléotidique choisie parmi SEQ ID N010, SEQ ID N011, SEQ ID N012, SEQ ID N040, SEQ ID N041, et leurs séquences complémentaires, chacun desdits fragments étant notamment une sonde, pour obtenir une composition diagnostique, prophylactique ou thérapeutique pour détecter, prévenir ou traiter une infection par un premier agent pathogène et/ou infectant, et un second agent pathogène et/ou infectant, associés à la polyarthrite rhumatoïde, ou une réactivation de l'un ou l'autre dudit premier agent et dudit second agent.

24. Utilisation d'une association comprenant un premier polypeptide codé de manière partielle ou totale par le premier fragment nucléotidique défini à la revendication 23, et un second polypeptide codé de manière partielle ou totale par le deuxième fragment nucléotidique défini à la revendication 23, pour obtenir une composition diagnostique, prophylactique ou thérapeutique pour détecter prévenir ou traiter une infection par un premier agent pathogène et/ou infectant, et un second agent pathogène et/ou infectant, associés à la polyarthrite rhumatoïde, ou une réactivation de l'un ou l'autre dudit premier agent et dudit second agent.

25. Utilisation selon la revendication 24, caractérisée en ce qu'elle comprend un premier ligand, notamment anticorps, spécifique du premier polypeptide, et un second ligand, notamment anticorps, spécifique du second polypeptide, lesdits premier et second polypeptides étant définis à la revendication 24.

26. Procédé d'obtention d'un premier agent pathogène et/ou infectant selon l'une des revendications 1 à 6 et/ou d'un second agent pathogène et/ou infectant selon l'une des revendications 13 ou 15, associés à la polyarthrite rhumatoïde, caractérisé en ce qu'on cultive in vitro des cellules de ponctions de liquide synovial notamment choisies parmi les synoviocytes et les fibroblastes desquamés de liquide articulaire, de patients atteints de polyarthrite rhumatoïde.

27. Procédé d'obtention d'un premier agent pathogène et/ou infectant selon l'une des revendications 1 à 6 et/ou d'un second agent pathogène et/ou infectant selon l'une des revendications 13 ou 14, associés à la polyarthrite rhumatoïde, caractérisé en ce qu'on cultive in vitro des cellules lymphocytaires B immortalisées par le virus d'Epstein-Barr, de patients atteints de polyarthrite rhumatoïde.

28. Fragment nucléotidique, dont la séquence nucléotidique comprend une séquence choisie parmi SEQ ID N039, SEQ ID N042, SEQ ID N043, leurs séquences complémentaires, et leurs séquences équivalentes, notamment les séquences nucléotidiques présentant pour toute suite de 100 monomères contigus, au moins 50 %, et de préférence au moins 70 % d'homologie, avec une séquence choisie parmi SEQ ID N039, SEQ ID N042, SEQ ID N043, et leurs séquences complémentaires.

29. Amorce spécifique pour l'amplification par polymérisation d'un ARN ou d'un ADN d'un matériel viral selon l'une quelconque des revendications 1 à 6, caractérisée en ce qu'elle comprend une séquence nucléotidique identique ou équivalente à au moins une partie de la séquence nucléotidique d'un fragment selon la revendication 28, notamment une séquence nucléotidique présentant, pour toute suite de 10 monomères contigus, au moins 70 % d'homologie avec au moins une partie dudit fragment.

30. Amorce selon la revendication 29, caractérisée en ce qu'elle comprend une séquence nucléotidique choisie parmi SEQ ID N047, SEQ ID N048, SEQ ID N049, et leurs séquences complémentaires.

31. Sonde susceptible de s'hybrider spécifiquement avec un ARN ou un ADN d'un matériel viral selon l'une quelconque des revendications 1 à 6, caractérisée en ce qu'elle comprend une séquence nucléotidique identique ou équivalente à au moins une partie de la séquence nucléotidique d'un fragment selon la revendication 28, notamment une séquence nucléotidique présentant, pour toute suite de 10 monomères contigus, au moins 70 % d'homologie avec au moins une partie dudit fragment.

32. Sonde selon la revendication 31, caractérisée en ce qu'elle comprend une séquence nucléotidique choisie parmi SEQ ID N047, SEQ ID N048, SEQ ID N049, et leurs séquences complémentaires.

33. Fragment nucléotidique, dont la séquence nucléotidique comprend une séquence choisie parmi SEQ ID N040, SEQ ID N041, leurs séquences complémentaires, et leurs séquences équivalentes, notamment les séquences nucléotidiques présentant pour toute suite de 100 monomères contigus, au moins 70 %, et de préférence au moins 90 % d'homologie, avec une séquence choisie parmi SEQ ID N040, SEQ ID N041, et leurs séquences complémentaires.

34. Amorce spécifique pour l'amplification par polymérisation d'un ARN ou d'un ADN d'un agent pathogène et/ou infectant selon l'une des revendications 12 à 14, comprenant une séquence nucléotidique identique ou équivalente à au moins une partie de la séquence nucléotidique d'un fragment selon la revendication 33, notamment une séquence nucléotidique présentant, pour toute suite de 10 monomères contigus, au moins 90 % d'homologie avec au moins une partie dudit fragment.

35. Amorce selon la revendication 34, caractérisée en ce qu'elle comprend une séquence nucléotidique choisie parmi SEQ ID N044, SEQ ID N045, SEQ ID N046, et leurs séquences complémentaires.

36. Sonde susceptible de s'hybrider spécifiquement avec un ARN ou un ADN d'un agent pathogène et/ou infectant selon l'une des revendications 12 à 14, comprenant une séquence nucléotidique identique ou équivalente à au moins une partie de la séquence nucléotidique d'un fragment selon la revendication 33, notamment une séquence nucléotidique présentant, pour toute suite de 10 monomères contigus, au moins 90 % d'homologie avec au moins une partie dudit fragment.

37. Sonde selon la revendication 36, caractérisée en ce qu'elle comprend une séquence nucléotidique choisie parmi SEQ ID N044, SEQ ID N045, SEQ ID N046, et leurs séquences complémentaires.
